# FASCICULE DE BREVET EUROPEEN

(11) **EP 2 800 756 B1**
(45) Date de publication et mention de la délivrance du brevet: **25.04.2018**
(21) Numéro de dépôt: 12819112.9
(22) Date de dépôt: 21.12.2012
(51) Int. Cl.: C07K 1/04, C07K 1/02

(54) **PEPTIDES C ALPHA-AMIDES, LEURS PROCÉDÉS DE PRÉPARATION ET LEURS UTILISATIONS COMME PRÉCURSEURS DE PEPTIDES C ALPHA-THIOESTERS POUR LA SYNTHÈSE DE PROTÉINES**
PEPTID-C-ALPHA-AMIDE, VERFAHREN ZUR HERSTELLUNG DAVON SOWIE ANWENDUNGEN DAVON ALS VORSTUFEN VON PEPTID-C-ALPHA-THIOESTERN FÜR PROTEINSYNTHESE
PEPTIDE C ALPHA-AMIDES, METHODS FOR PREPARING SAME AND USES THEREOF AS PRECURSORS OF PEPTIDE C ALPHA-THIOESTERS FOR PROTEIN SYNTHESIS

(30) Priorité: 03.01.2012 FR 1250060
(43) Date de publication de la demande: 12.11.2014
(73) Titulaire: Centre National de la Recherche Scientifique (C.N.R.S.), 75794 Paris Cedex 16 (FR)
(72) Inventeur: AUCAGNE, Vincent, F-45400 Fleury-les-Aubrais (FR); DELMAS, Agnès, F-45000 Orléans (FR); ADIHOU, Hélène, 77500 Chelles (FR)
(74) Mandataire: Lavoix
(86) Numéro de dépôt international: PCT/FR2012/053074
(87) Numéro de publication internationale: WO 2013/102723

(56) Documents cités:
- WO-A2-2006/013209
- US-A1- 2011 184 148
- KOHEI SATO ET AL: "N-Sulfanylethylanilide Peptide as a Crypto-Thioester Peptide", CHEMBIOCHEM, vol. 12, no. 12, 16 août 2011 (2011-08-16) , pages 1840-1844, XP055040434, ISSN: 1439-4227, DOI: 10.1002/cbic.201100241
- WEN HOU ET AL: "Peptidyl N , N -Bis(2-mercaptoethyl)-amides as Thioester Precursors for Native Chemical Ligation +", ORGANIC LETTERS, vol. 13, no. 3, 4 février 2011 (2011-02-04), pages 386-389, XP055040436, ISSN: 1523-7060, DOI: 10.1021/ol102735k cité dans la demande

## Description

La présente invention concerne de nouveaux peptides C^{α}-amides dont l'intérêt repose sur le fait que ce sont des précurseurs directs de peptides C^{α}-thioesters, les procédés de préparation desdits peptides et leurs utilisations pour la synthèse de protéines, notamment de protéines d'intérêts thérapeutiques.

Les protéines sont des macromolécules biologiques ubiquitaires dans le vivant et présentant pour certaines un fort potentiel thérapeutique. Il s'agit d'oligomères linéaires d'acides α-aminés (notés H-Xaa-OH) liés par des liaisons amides. Les petites protéines (moins d'une cinquantaine de résidus Xaa) sont appelées peptides. Afin de pouvoir étudier leurs possibles applications thérapeutiques il est capital de pouvoir les produire aisément en quantité suffisante. Les protéines nécessaires à ces études peuvent être obtenues par deux voies. La méthode biotechnologique, qui fait intervenir la technique dite de l'ADN recombinant, est souvent utilisée mais présente de nombreuses limitations. La synthèse par voie chimique est une technique complémentaire très intéressante pour la production de protéines comportant jusqu'à plus de deux cent résidus d'acides α-aminés. Elle permet d'accéder à des protéines difficiles à obtenir par voie biotechnologique, comme les protéines cytotoxiques ou celles modifiées spécifiquement, à la fois sur les chaînes latérales des résidus introduits (modifications post-traductionnelles, sondes, chaînes de polyéthylèneglycols...) mais également sur le squelette peptidique et les extrémités N- et C-terminales.

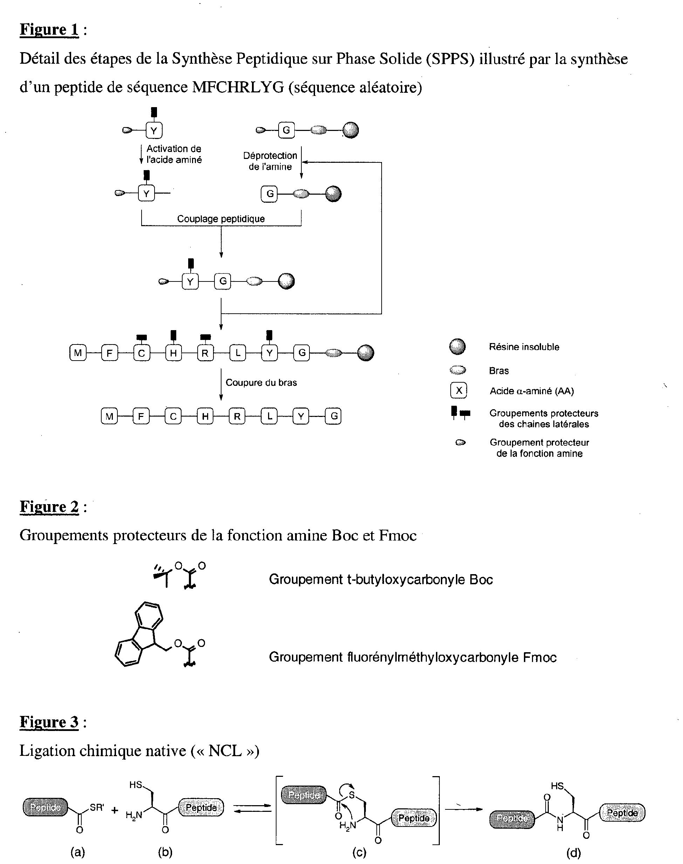

La synthèse chimique de petites protéines peut être effectuée par Synthèse Peptidique sur Phase Solide **(SPPS)** (Merrifield, B. Solid phase synthesis, Science 1986, 232, 341-347). La SPPS est une technique qui permet, par le biais d'un «bras», de fixer un acide aminé C-terminal muni de groupements protecteurs adaptés sur un support solide, généralement un polymère insoluble appelé « résine ». Les acides aminés suivants sont ajoutés un par un jusqu'à atteindre le N-terminus, par une succession de réactions chimiques de couplage et déprotection. De cette façon, il est possible d'utiliser de très larges excès de réactifs, ce qui augmente considérablement le rendement des réactions. La seule purification qui devient alors nécessaire est un rinçage abondant de la résine effectué par simple filtration après chaque réaction, les étapes de purification chromatographique intermédiaires étant supprimées. Le bras est inerte dans toutes les conditions de la synthèse et permet la libération du peptide en solution uniquement lors d'une étape finale de coupure du bras, habituellement concomitante avec la déprotection des chaînes latérales. Le détail des étapes de la Synthèse Peptidique sur Phase Solide (SPPS) est illustré à la figure 1.

Deux stratégies principales de synthèse ont été développées en SPPS, à savoir la SPPS en stratégie Boc ou la SPPS en stratégie Fmoc, qui mettent en jeu respectivement des acides α-aminés *N^{α}-*protégés soit par un groupement *t-*butyloxycarbonyle (Boc) soit par un groupement fluorénylméthyloxycarbonyle (Fmoc). Ces deux groupes protecteurs Boc et Fmoc sont illustrés à la figure 2. La SPPS en stratégie Boc nécessite un traitement final au fluorure d'hydrogène (HF) dangereux et délicat à mettre en oeuvre, et est de ce fait de plus en plus délaissée. La SPPS en stratégie Fmoc **(Fmoc SPPS)** est la plus largement utilisée de nos jours.

La SPPS est limitée en termes de taille des protéines synthétisable en routine (< ∼50 résidus d'acides aminés). Une méthode alternative consiste à synthétiser des fragments de protéines *via* la SPPS qui peuvent être condensés grâce à des réactions hautement chimiosélectives. Ces réactions se réalisent généralement en milieu aqueux tamponné entre des partenaires peptidiques déprotégés et sont appelées ligations chimiques. Lorsque la liaison qui résulte de la ligation est une liaison amide, la ligation chimique est dite « native » (Dawson P. et al.; Synthesis of proteins by native chemical ligation, Science 1994, 266, 776-779). La technique de ligation chimique native « **Native Chemical Ligation : NCL** » la plus utilisée est illustrée à la figure 3. Cette réaction est au jour d'aujourd'hui la méthode de référence pour la synthèse chimique de protéines. Cette technique fait intervenir un peptide dont le C-terminus est modifié par un C^{α}-thioester (voir composé nommé (a) sur la figure 3 avec R' représentant un radical provenant d'un thiol de formule R'-SH) et un peptide portant une cystéine N-terminale (voir composé nommé (b)). Dans un premier temps, le groupement sulfhydrile de la cystéine N-terminale du composé (b) réagit par une réaction de *trans*-thioestérification avec la fonction thioester de (a). Le composé (c) qui en résulte se réarrange spontanément par un transfert d'acyle du soufre vers l'azote (S→N) *via* un état de transition cyclique à cinq chaînons, thermodynamiquement très favorisé. On obtient ainsi un peptide (d) présentant une liaison amide à la jonction entre les deux fragments.

Contrairement à la synthèse de peptides C^{α}-acides et C^{α}-amides, les peptides C^{α}-thioesters sont particulièrement difficiles à synthétiser par SPPS en stratégie Fmoc du fait de la non-compatibilité de la fonction thioester (-CO-S-) avec la pipéridine utilisée pour déprotéger le groupement Fmoc. La pipéridine est en effet une amine nucléophile qui réagit rapidement avec les fonctions thioesters pour couper la liaison carbone-soufre et donner l'amide et le thiol correspondants. Les stratégies contemporaines pour la synthèse de peptides thioesters par Fmoc SPPS sont pour leur vaste majorité des méthodes indirectes : la fonction thioester n'est introduite qu'après avoir réalisé l'élongation complète du peptide.

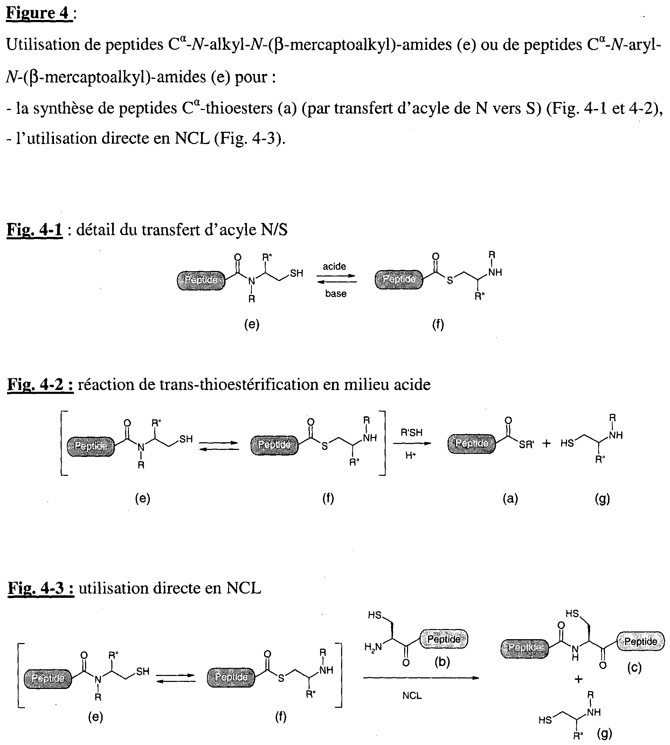

Parmi les différentes méthodes existantes permettant de synthétiser des peptides C^{α}-thioesters par SPPS en stratégie Fmoc (figure 4-2, composé (a)), certaines font intervenir des peptides C^{α}-*N-*alkyl-*N*-(β-mercaptoalkyl)-amides (figure 4-1, composé (e) avec R = alkyle et R" représentant un groupe comprenant une fonction ayant permis, lors des premières étapes de la synthèse de (e), de greffer un précurseur de (e) à un polymère insoluble par l'intermédiaire d'un bras) ou des peptides C^{α}-*N-*aryl-*N-*(β-mercaptoalkyl)-amides (figure 4-1, composé (e) avec R = aryle et R" tel que défini précédemment).

De tels peptides C^{a}-β-mercapto-amides (e) sont généralement spontanément en équilibre avec une forme C^{a}-β-amino-thioester (f) *via* un transfert d'acyle intramoléculaire de l'azote vers le soufre (N→S) détaillé dans la figure 4-1. L'équilibre est déplacé vers la forme amide (e) dans des conditions neutres ou basiques, alors que la forme thioester (f) prédomine en milieu acide. La réaction avec un excès de thiol (R'SH) à pH acide de composés de type (e) ou (f) permet de déplacer progressivement cet équilibre et d'obtenir le peptide C^{α}-thioester (a) dénué de fonction β-amine (composé (a)).

Il a été montré dans plusieurs exemples récents que certains de ces peptides C^{α}-β-mercapto-amides (e) peuvent être directement utilisés, au même titre que les peptides C^{α}-thioesters (f), dans les conditions de NCL, c'est-à-dire en présence d'un peptide muni d'une cystéine N-terminale de type (b) et d'un excès de thiol R'SH, dans un milieu tamponné à un pH proche de la neutralité. Le principe est *a priori* que le transfert d'acyle N→S et la trans-thioestérification se font *in situ* lors de la ligation (figure 4-3). Les peptides C^{α}-β-mercapto-amides (e) dotés de cette propriété sont appelés « crypto-thioester ». Seuls quelques exemples ont été décrits à ce jour (1-Ollivier N. et al.; Bis(2-sulfanylethyl)amino native peptide ligation, Org. Lett. 2010, 12, 5238-5241; 2- Sato K. et al.; N-Sulfanylethylanilide peptide as a crypto-thioester peptide, ChemBioChem 2011, 12, 1840-1844; 3- Hou W. et al.; Peptidyl N,N-bis(2-mercaptoethyl)-amides as thioester precursors for native chemical ligation. Organic Letters 2011, 13,386-389).

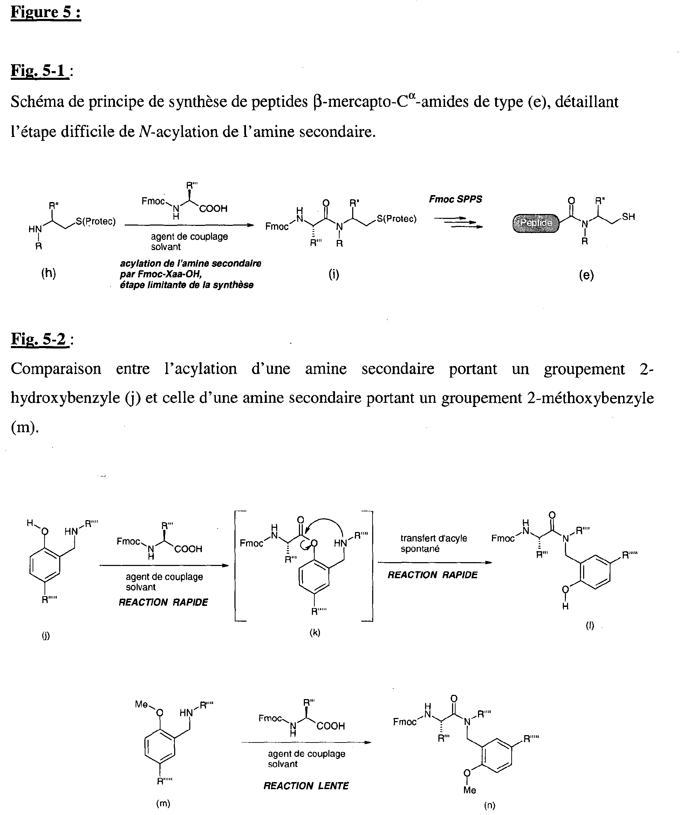

La synthèse de peptides peptides C^{α}-*N-*alkyl-*N-*(β-mercaptoalkyl)-amides ou C^{α}-*N*-aryl-*N*-(β-mercaptoalkyl)-amides (voir figure 5-1, composé (e) avec R = alkyl ou aryl) se réalise par SPPS en stratégie Fmoc en masquant la fonction thiol du composé (h) par un groupement protecteur (Protec), ce qui a pour conséquence d'empêcher le réarrangement en une forme thioester de type (f) (figure 4-3). Le peptide reste sous sa forme amide, et est donc stable tout au long de l'élongation.

Le principal inconvénient de cette approche de la synthèse de thioesters ou de crypto-thioesters réside dans le fait que toutes les synthèses décrites à ce jour ont en commun une première étape extrêmement délicate de *N*-acylation (figure 5-1). En effet, à cause de l'encombrement stérique autour de l'atome d'azote, la cinétique de la réaction est généralement très lente ce qui peut conduire à une *N*-acylation incomplète, des produits secondaires indésirables et des rendements faibles. Dans de nombreux cas, cette approche est limitée en pratique à des peptides présentant un résidu glycine en C-terminal.

Il subsiste donc toujours le besoin de synthétiser des peptides C^{α}-*N*-alkyl-*N*-(β-mercaptoalkyl)-amides ou C^{α}-*N*-aryl-*N*-(β-mercaptoalkyl)-amides par une méthode simple et efficace. En effet, une préparation simple et efficace de tels peptides C^{α}-amides permet notamment une préparation simple et efficace des peptides C^{α}-thioesters puisque ces peptides C^{α}-amides sont des précurseurs directs des peptides C^{α}-thioesters.

En outre, de tels peptides C^{α}-amides pourront également être dotés de propriétés de crypto-thioester, et être utilisés directement dans une réaction de NCL sans qu'il soit nécessaire d'isoler au préalable la forme thioester.

De façon avantageuse selon l'invention, la *N*-acylation de l'amine secondaire de type (h) (figure 5-1) est avantageusement accélérée notamment grâce à un radical R tout à fait original permettant une assistance intramoléculaire lors de la *N*-acylation.

Le principe d'une telle assistance de l'acylation d'une amine secondaire a été décrit dans la littérature mais jamais appliqué à des peptides C^{α}-β-mercapto-amides précurseurs de C^{α}-thioesters. A titre d'exemple, on pourra citer l'assistance de l'acylation d'une amine secondaire d'un peptide par une fonction *N*-(2-hydroxybenzyle) (Johnson T.et al.; A reversible protecting group for the amide bond in peptides. Use in the synthesis of "difficult sequences", J. Chem. Soc. Chem. Commun., 1993, 369-374.).

Cette assistance est illustrée dans la figure 5-2. La *N*-acylation de l'amine secondaire (j) est beaucoup plus rapide que celle de l'amine secondaire (m), alors que l'encombrement stérique autour de l'atome d'azote (principal facteur déterminant la cinétique d'une réaction de *N*-acylation classique) de chacun de ces deux composés est comparable. Cette grande différence de réactivité est due à la présence d'un groupement *N-*(2-hydroxybenzyle) dans (j) mais pas dans (m). La fonction hydroxyle de ce groupe peut être *O*-acylée dans des conditions de *N-*acylation, pour donner un intermédiaire (k). Contrairement à la fonction amine secondaire, la fonction hydroxyle est très peu encombrée, et cette réaction de *O-*acylation est rapide. L'intermédiaire (k) va rapidement subir un transfert d'acyle de l'oxygène vers l'azote (O→N) pour donner l'amide (l) *via* un état de transition cyclique à six chaînons, thermodynamiquement très favorisé. Cette *N*-acylation par transfert d'acyle intramoléculaire conduisant à (l) est très peu sensible à l'encombrement stérique autour de l'atome d'azote, contrairement à la version intermoléculaire conduisant à (n), ce qui explique la *N*-acylation beaucoup plus rapide de (j) comparé à (m). Une assistance similaire a également été évoquée lors de l'acylation de peptides munis d'une fonction amine muni d'un groupement de type γ-(2-aza-hétérocycle) (Zhang L. et al.; Orthogonal coupling of unprotected peptide segments through histidyl amino terminus, Tetrahedron Lett. 1997, 38, 3-6.).

L'un des buts de la présente invention est de proposer une méthode de synthèse de peptides C^{α}-*N*-alkyl-*N*-(β-mercaptoalkyl)-amides précurseurs de peptides C^{α}-thioesters qui soit à la fois simple à mettre en oeuvre, rapide, non-onéreuse et qui ne génère pas de produits secondaires indésirables.

De façon avantageuse selon l'invention, certains des groupements présents sur les radicaux R originaux tels que mentionnés précédemment pourront permettre également une assistance intramoléculaire du transfert d'acyle N→S et/ou de la *trans*-thioesterification, et ainsi augmenter l'aptitude des peptides C^{α}-*N-*alkyl-*N*-(β-mercaptoalkyl)-amides de l'invention à se comporter comme des crypto-thioesters. Cette assistance pourra résulter par exemple de la formation de liaisons hydrogènes, d'interactions dipolaires, d'interactions π-π, ou d'une catalyse acidobasique.

Un autre but de l'invention est de proposer une méthode de synthèse de peptides C^{α}-*N*-alkyl-*N-*(β-mercaptoalkyl)-amides originaux qui soient dotés de propriétés de crypto-thioesters afin de pouvoir être directement utilisés comme partenaire dans des réactions de NCL.

Dans la présente demande, les peptides C^{α}-*N*-alkyl-*N*-(β-mercaptoalkyl)-amides seront plus simplement dénommés peptides C^{α}-amides. Les peptides C^{α}-amides de l'invention couvrent à la fois lesdits peptides C^{α}-*N*-alkyl-*N*-(β-mercaptoalkyl)-amides mais aussi des peptides C^{α}-*N-*alkyl-*N-*(γ-mercaptoalkyl)-amides, C^{α}-*N*-alkyl-*N-*(β-sélénoalkyl)-amides et C^{α}-*N*-alkyl-*N*-(γ-sélénoalkyl)-amides, lesdits peptides C^{α}-amides de l'invention étant des précurseurs de peptides C^{α}-thioesters, selon un mécanisme supposé de transfert d'acyle du soufre ou du sélénium vers l'azote.

Les peptides C^{α}-amides de l'invention peuvent aussi être dénommés peptides « crypto-thiosters » du fait de la possibilité de les utiliser directement dans des réactions de NCL, sans qu'il soit nécessaire de préparer au préalable la forme thioester.

La présente invention a ainsi pour objet un peptide C^{α}-amide précurseur d'un peptide C^{α}-thioester caractérisé en ce qu'il comprend le radical de formule générale (I): 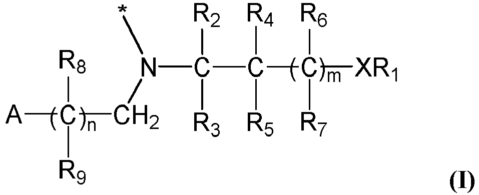 dans laquelle
X représente un atome de soufre ou de sélénium,
R₁ représente un atome d'hydrogène ou un groupe protecteur du soufre ou du sélénium compatible avec des conditions d'élongation par Fmoc SPPS,
m représente un entier égal à 0 ou 1,
un des R₂, R₃, R₄, R₅, R₆ ou R₇ représente le radical -B-C-D dans lequel :
   D représente un atome d'hydrogène ou un support solide convenant pour la synthèse de peptides en phase solide (SPPS),
   C est absent ou représente un bras utilisable pour la SPPS,
   B représente un radical divalent comportant un hétéroatome,
les autres desdits R₂, R₃, R₄, R₅, R₆ ou R₇ qui ne représentent pas -B-C-D représentent alors indépendamment l'un de l'autre un atome d'hydrogène, un radical alkyle ayant de 1 à 10 atomes de carbone, et de préférence un radical méthyle (-CH₃) ou un radical phényle (-C₆H₅), à la condition qu'au plus deux desdits radicaux R₂, R₃, R₄, R₅, R₆ ou R₇ représentent en même temps un phényle,
R₈ et R₉ représentent indépendamment l'un de l'autre un radical alkyle ayant de 1 à 10 atomes de carbone, et de préférence un radical méthyle ou un atome d'hydrogène,
n représente un entier allant de 0 à 4, de préférence de 0 à 1,
A représentant un radical aryle ou hétéroaryle choisi dans le groupe comprenant
   - le radical de formule : 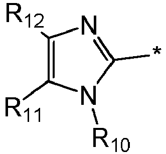 dans laquelle
      R₁₀ représente un radical alkyle ayant de 1 à 10 atomes de carbone, et de préférence un méthyle,
      R₁₁ et R₁₂ représentent indépendamment l'un de l'autre un atome d'hydrogène, un atome d'halogène choisi dans le groupe comprenant Cl, Br, I ou F, un radical -CN, un radical -NO₂, un radical -CF₃, un radical phényle (-C₆H₅), un radical -CONH₂, un radical R₁₀, un radical -OR₁₀, un radical -SR₁₀, un radical -N(R₁₀)₂, un radical -COOR₁₀, un radical -CONHR₁₀ ou un radical -CON(R₁₀)₂, R₁₀ étant tel que défini précédemment,
   - le radical de formule : 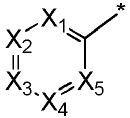 dans laquelle
      au moins un des X₁, X₂, X₃, X₄ et X₅ représente un atome d'azote (N), un radical C-OH ou un radical C-SH, les autres desdits X₁, X₂, X₃, X₄ ou X₅ qui ne représentent pas N, C-OH ou C-SH représentent alors indépendamment l'un de l'autre un radical C-R₁₁ avec R₁₁ tel que défini précédemment,
   - le radical de formule : 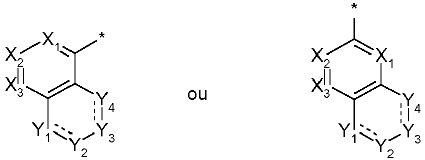 dans laquelle
      au moins un des X₁, X₂, X₃ et Y₄ représente un atome d'azote (N), un radical C-OH ou un radical C-SH, les autres desdits X₁, X₂ ou X₃ qui ne représentent pas N, C-OH ou C-SH représentent alors indépendamment l'un de l'autre un radical C-R₁₁ avec R₁₁ tel que défini précédemment,
      Y₁, Y₂, Y₃ et Y₄ (à condition que Y₄ ne représente pas C-OH ou C-SH) représentent indépendamment l'un de l'autre, selon qu'ils soient liés par une liaison simple ou double, un atome d'azote (N) ou un groupe NR₁₀ avec R₁₀ tel que défini précédemment, un groupe CH ou CH₂, un groupe C-R₁₁ ou CHR₁₁ ou CR₁₁R₁₂ avec R₁₁ et R₁₂ tels que définis précédemment, un carbonyle (C=O), un atome d'oxygène (O), un atome de soufre (S), avec la condition qu'au plus deux desdits Y₁, Y₂, Y₃ et Y₄ représentent en même temps un atome d'oxygène ou un atome de soufre,
      l'un desdits Y₁, Y₂, Y₃ ou Y₄ pouvant être absent, de sorte à former un cycle à 5 chaînons.

Par alkyle on entend un radical hydrocarbyle ayant 1 à 10 atomes de carbone, répondant à la formule générale CₙH₂ₙ₊₁ où n est supérieur ou égal à 1. Les groupes alkyles peuvent être linéaires ou ramifiés et peuvent être substitués par les groupements indiqués dans la présente demande.

Par aryle on entend un groupe hydrocarbyle polyinsaturé, aromatique, ayant un seul cycle (comme par exemple le phényle) ou plusieurs cycles accolés (comme par exemple le naphtyle) ou plusieurs cycles reliés par une liaison covalente (par exemple biphényle), qui contiennent typiquement 5 à 12 atomes de carbone, préférentiellement 6 ou 12, et où au moins un cycle est aromatique. Le cycle aromatique peut optionnellement comprendre un à deux cycles supplémentaires (soit cycloalkyle, hétérocycloalkyle ou hétéroaryle) accolés. Le terme aryle comprend également les dérivés partiellement hydrogénés de systèmes carbocycliques décrits ci-dessus. Le cycle aromatique peut optionnellement comprendre un à cinq substituants (différents de H) de type alkyle (préférentiellement méthyle), -F, -Cl, - Br, -I, -CF₃, -OMe, -SMe, -N(Me)₂, -COOH, -SO₃H, -CH₂N(Me)₂, -CH₂COOH, - CH₂SO₃H, -CH₂CH₂N(Me)₂, -CH₂CH₂COOH, -CH₂CH₂SO₃H, -OCH₂COOH, - OCH₂SO₃H, -OCH₂CH₂N(Me)₂, -OCH₂CH₂COOH ou -OCH₂CH₂SO₃H.

Par hétéroaryle on entend un cycle ou plusieurs cycles accolés ou reliés par une liaison covalente, comprenant 3 à 17 atomes de carbone, préférentiellement 3 à 11 atomes de carbone, où au moins l'un des cycles est aromatique et où au moins un ou plusieurs atomes de carbone sont remplacés par de l'oxygène, de l'azote et/ou du soufre. Le terme hétéroaryle comprend également des systèmes décrits ci-dessus ayant un groupe aryle, cycloalkyle, hétéroaryle ou hétérocycloalkyle accolé ou un à cinq substituants alkyle, préférentiellement méthyle. Le terme hétéroaryle comprend également des systèmes décrits ci-dessus comportant un à quatre substituants (différents de H) de type alkyle (préférentiellement méthyle), -F, -Cl, -Br, -I, -CF₃, - OMe, -SMe, -N(Me)₂, -COOH, -SO₃H, -CH₂N(Me)₂, -CH₂COOH, -CH₂SO₃H, - CH₂CH₂N(Me)₂, -CH₂CH₂COOH, -CH₂CH₂SO₃H, -OCH₂COOH, -OCH₂SO₃H, - OCH₂CH₂N(Me)₂, -OCH₂CH₂COOH ou -OCH₂CH₂SO₃H.

Par cycloalkyle on entend un hydrocarbyle monovalent, cyclique, insaturé ou saturé, présentant un ou deux cycles et comportant 3 à 10 atomes de carbone.

Par hétérocycloalkyle on entend un cycloalkyle dans lequel au moins un atome de carbone est remplacé par un atome d'oxygène, d'azote et/ou de soufre.

Selon un mode de réalisation avantageux de l'invention, le radical divalent B présent dans le radical (I), représente : 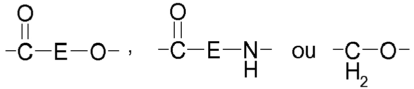 dans lequel
E est absent ou représente le groupe -(Xaa)ᵢ- dans lequel :
   i représente un entier allant de 1 à 20, et de préférence de 1 à 5,
   chaque Xaa représente indépendamment les uns des autres un résidu d'acide aminé, et lorsque i est supérieur ou égal à 2, chacun desdits Xaa est relié à son voisin Xaa par une liaison peptidique,
   ou, dans le cas où m =1, et R₄ ou R₅ = -B-C-D, alors le radical divalent B représente de préférence : 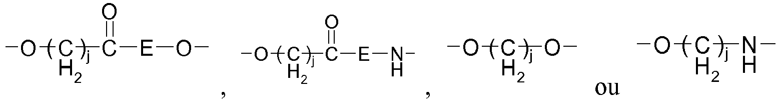 dans lequel :
   j représente un entier allant de 0 à 10, de préférence de 0 à 3 et E est tel que défini précédemment.

Les éventuelles fonctions réactives des chaînes latérales desdits résidus d'acides aminés peuvent être libres c'est-à-dire non protégées ou au contraire protégées par des groupes protecteurs habituellement utilisés par l'homme de l'art lors de la SPPS.

Selon un autre mode de réalisation avantageux de l'invention, dans le radical (I), C représente un bras utilisable pour la SPPS en stratégie Fmoc, et de préférence un bras acido-labile choisi dans le groupe comprenant un bras de Rink (4-[(2,4-diméthoxyphényl)méthyl]phénoxyacétyle), un bras de type Wang (4-alkoxybenzyle), un bras de Sieber (xanthén-3-yloxyalkyle), un bras PAL (4-2,5-diméthoxy-alkoxybenzyle), un bras 2-chlorotrityle, un bras PAM (phénylacetamidométhyle), un bras SASRIN (2-méthoxy-4-alkoxybenzyle) ou un bras MBHA (4-méthyl)benzhydryle.

Selon un autre mode de réalisation avantageux de l'invention, dans le radical (I), D représente un support solide convenant pour la SPPS en stratégie Fmoc et de préférence est choisi dans le groupe comprenant une résine commercialisée sous la dénomination PEGA®, une résine commercialisée sous la dénomination CHEMMATRIX™, une résine de type polyacrylamide, une résine de type polystyrène ou une résine mixte polystyrène/polyéthylèneglycol (PEG).

A titre d'exemple de résine mixte polystyrène/polyéthylèneglycol (PEG), on pourra citer celles commercialisées sous la dénomination tentagel ou argogel.

Selon un mode de réalisation avantageux de l'invention, dans le radical (I), n est un entier égal à 0, et le radical A présente la formule : 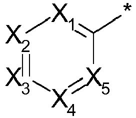 dans laquelle X₁ représente C-OH, et chacun des X₂, X₃, X₄ et X₅ est tel que défini précédemment.

De manière préférée, chacun des X₂, X₃ ou X₅ représente CH et X₄ représente C-R₁₁ avec R₁₁ tel que défini précédemment.

De manière encore plus préférée X₄ représente CH, C-OMe ou C-NO₂.

Ainsi un radical A préféré est celui représenté par l'une des formules suivantes :

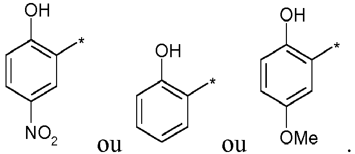

Selon un autre mode de réalisation avantageux de l'invention, dans le radical (I), n est un entier égal à 0, et le radical A présente la formule : 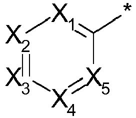 dans laquelle X₁ représente l'atome d'azote (N) et chacun desdits X₂, X₃, X₄ et X₅ est tel que défini précédemment. De manière préférée, chacun des X₂, X₃ ou X₅ représente CH et X₄ représente CH ou C-R₁₁ avec R₁₁ représentant OCH₃ ou N(CH₃)₂.

Ainsi un autre radical A préféré est celui représenté par l'une des formules suivantes :

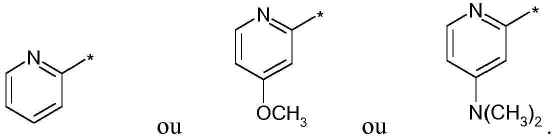

Selon un autre mode de réalisation avantageux de l'invention, dans le radical (I), n est un entier égal à 1, et le radical A présente la formule : 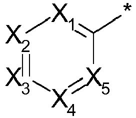 dans laquelle X₁ représente l'atome d'azote (N) et chacun desdits X₂, X₃, X₄ et X₅ est tel que défini précédemment. 9. De manière préférée, chacun des X₂, X₃, X₄ et X₅ représente CH.

Ainsi un autre radical A préféré avec n égal à 1 est celui représenté par la formule suivante :

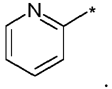

Selon un autre mode de réalisation avantageux de l'invention, dans le radical (I), n est un entier égal à 0, et le radical A présente la formule : 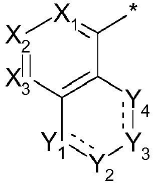 dans laquelle X₁ ou Y₄ représente C-OH ou N, l'autre desdits X₁ ou Y₄ qui ne représente pas C-OH ou N, ainsi que chacun desdits X₂, X₃, Y₁, Y₂, et Y₃ sont tels que définis précédemment.

Selon un autre mode de réalisation avantageux de l'invention, dans le radical (I), n est un entier égal à 0, et le radical A présente la formule : 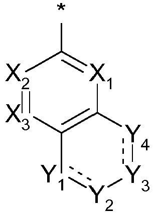 dans laquelle X₁ ou X₂ représente C-OH ou N, l'autre desdits X₁ ou X₂ qui ne représente pas C-OH ou N, ainsi que chacun desdits X₃, Y₁, Y₂, Y₃ et Y₄ sont tels que définis précédemment.

Selon un mode de réalisation préféré de l'invention, le peptide C^{α}-amide est caractérisé en ce que dans le radical (I) :
m représente 0 ou 1,
chacun desdits R₃, R₄, R₅, R₆ ou R₇ représente H,
R₂ représente -B-C-D dans lequel B représente : 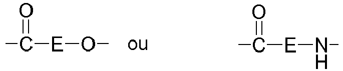
et C, D et E sont tels que définis précédemment.

Selon un mode de réalisation préféré de l'invention, dans le radical (I), m est égal à 0 et X représente un atome de soufre (S).

Lorsque R₁ est un groupe protecteur du soufre, alors ce dernier peut être choisi dans le groupe comprenant trityle (Trt), méthyltrityle (Mtt), méthoxytrityle (Mmt), xanthényl (Xan), tri-méthoxybenzyle (Tmob), acétamidométhyle (Acm), triméthylacétamidométhyle (Tacm), benzamidométhyle (Bam), allyloxycarbonylaminométhyle (Allocam), phtalimidométhyle (Pim), 2-(triméthylsilyl)éthyle, t-butyle (tBu), 2,4,6-triméthylbenzyle, quinolinylméthyle (Qm), diphényl-4-pyridylméthyle, 1-adamantyle, benzyloxyméthyle (BOM), 2-tétrahydropyranyle (Thp), benzylthiométhyle, éthylsulfényle (SEt), t-butylsulfényle (StBu), phénylsulfényle (SPh) et 2,4-dinitrophényle.

Plus particulièrement, selon un mode de réalisation avantageux de l'invention, lorsque R₁ est un groupe protecteur du soufre pouvant être coupé par un traitement à l'acide trifluoroacétique (TFA), alors ce dernier est de préférence choisi dans le groupe comprenant trityle (Trt), méthyltrityle (Mtt), méthoxytrityle (Mmt), xanthényl (Xan) et tri-méthoxybenzyle (Tmob).

Selon un autre mode de réalisation avantageux de l'invention, lorsque R₁ est un groupe protecteur du soufre stable à un traitement au TFA et stable dans des conditions de NCL, alors ce dernier est de préférence choisi dans le groupe comprenant acétamidométhyle (Acm), triméthylacétamidométhyle (Tacm), benzamidométhyle (Bam), allyloxycarbonylaminométhyle (Allocam), phtalimidométhyle (Pim), 2-(triméthylsilyl)éthyle, t-butyle (tBu), 2,4,6-triméthylbenzyle, quinolinylméthyle (Qm), diphényl-4-pyridylméthyle, 1-adamantyle, benzyloxyméthyle (BOM), 2-tétrahydropyranyle (Thp) et benzylthiométhyle.

Selon encore un autre mode de réalisation avantageux de l'invention, lorsque R₁ est un groupe protecteur du soufre qui est stable à un traitement au TFA mais labile dans des conditions de NCL, alors ce dernier est de préférence choisi dans le groupe comprenant éthylsulfényle (SEt), t-butylsulfényle (StBu), phénylsulfényle (SPh) et 2,4-dinitrophényle.

Selon encore un autre mode de réalisation avantageux, R₁ est un groupement labile dans une réaction de ligation chimique native NCL et est de préférence choisi dans le groupe contenant les groupements éthylsulfényle (SEt), t-butylsulfényle (StBu) et phénylsulfényle (SPh).

Selon un mode de réalisation avantageux de l'invention, X représente un atome de sélénium (Se), R₁ est un groupe protecteur du sélénium et est de préférence choisi dans le groupe comprenant 4-méthoxybenzyle (Mob), 4-méthylbenzyle (MeBzl) ou benzyle (Bzl).

A titre d'exemple de radical (I) selon l'invention, on pourra citer un de ceux choisis dans le groupe comprenant : 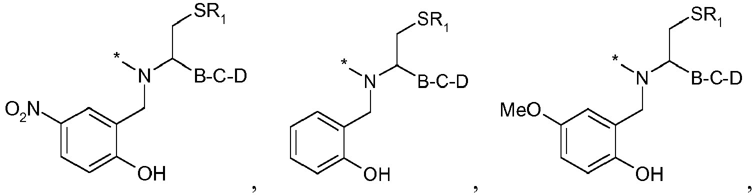 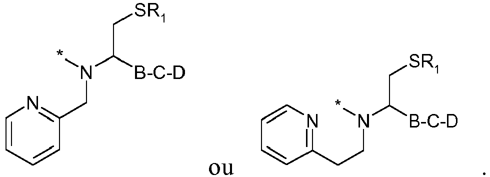 dans lequel :
B représente 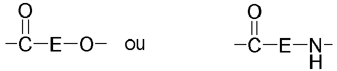 et E, C, D et R₁ sont tels que définis précédemment.

Le peptide C^{α}-amide objet de l'invention est plus particulièrement caractérisé en ce qu'il présente la formule générale (II) : 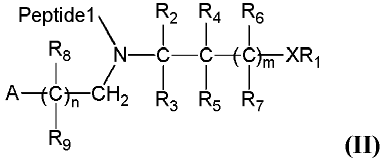 dans laquelle :
Peptide1 représente le groupement R₁₈-(Xaa)ₖ- dans lequel :
   k est un entier allant de 1 à 100, de préférence de 4 à 60, et plus préférentiellement encore de 8 à 40,
   Xaa représente indépendamment les uns des autres un résidu d'acide aminé provenant d'un acide aminé de formule H-Xaa-OH, et lorsque k est supérieur ou égal à 2, chacun desdits Xaa est relié à son voisin Xaa par une liaison peptidique,
   R₁₈ est un atome d'hydrogène ou un substituant de l'extrémité N-terminale comprise dans le résidu Xaa,
   X, m, n, A et R₁ à R₉ étant tels que définis précédemment.

De manière préférée, dans le radical divalent B tel que défini ci-dessus, E est absent ou représente -(Xaa)ᵢ- qui est choisi de manière à augmenter la solubilité aqueuse du Peptide1.

Les éventuelles fonctions réactives des chaînes latérales des résidus d'acides aminés -(Xaa)ₖ- peuvent être libres c'est-à-dire non protégées ou au contraire protégées par des groupes protecteurs habituellement utilisés par l'homme de l'art lors de la SPPS.

Selon un premier mode de réalisation avantageux de l'invention, le procédé de préparation du composé de formule (I bis) (ledit composé (I bis) formant le radical (I) au sein du peptide C^{α}-amide de l'invention) est caractérisé en ce qu'il comporte :
- une étape de greffage, sur un support solide de formule :

   H-E-O-C-D', H-E-NH-C-D' ou Cl-C-D'

   dans lequel E et C tels que définis précédemment, D' représente une résine convenant pour la SPPS et H, O, NH et Cl correspondant aux symboles chimiques habituellement utilisés,
   d'un composé de formule générale (III) : 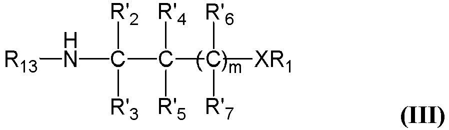 dans laquelle :
   R₁₃ représente un groupement protecteur de la fonction amine, et de préférence un groupement Fmoc,
   m, X et R₁ sont tels que définis précédemment,
   un des R'₂, R'₃, R'₄, R'₅, R'₆ ou R'₇ représentant un groupe réactif comprenant une fonction chimique réactive choisie de préférence dans le groupe comprenant -COOH, -NH₂ ou -OH, et permettant de greffer ledit composé (III) sur le support solide tel que mentionné ci-dessus selon une méthode connue de l'homme de l'art pour la SPPS en stratégie Fmoc,
   les autres desdits R'₂, R'₃, R'₄, R'₅, R'₆ ou R'₇ qui ne représentent pas ledit groupe réactif ont la même signification que lesdits R₂, R₃, R₄, R₅, R₆ ou R₇ tels que définis précédemment et ne représentent pas -B-C-D,
   afin d'obtenir un composé de formule générale (IV) : 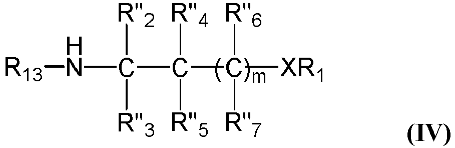
   dans laquelle :
   m, X, R₁ et R₁₃ sont tels que définis précédemment,
   un des R"₂, R"₃, R"₄, R"₅, R"₆ ou R"₇ représente -B-C-D' dans lequel B, C et D' sont tels que définis précédemment,
   les autres desdits R"₂, R"₃, R"₄, R"₅, R"₆ ou R"₇ qui ne représentent pas le radical -B-C-D' ont la même signification que lesdits R₂, R₃, R₄, R₅, R₆ ou R₇ tels que définis précédemment et ne représentent pas le radical -B-C-D,
- ladite étape de greffage étant suivie d'une étape de coupure du groupement protecteur R₁₃ du composé (IV) afin d'obtenir un composé de formule générale (IV bis) : 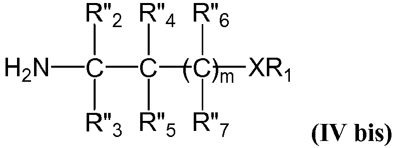 dans laquelle :
   m, X, R₁, R"₂, R"₃, R"₄, R"₅, R"₆ et R"₇ sont tels que définis précédemment,
- ladite étape de coupure étant suivie d'une étape de mono-*N-*alkylation du composé (IV bis) afin d'obtenir le composé de formule générale (V) : 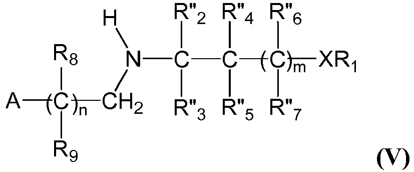
   dans laquelle X, R₁, m, R"2, R"₇ R"₄, R"₅, R"₆, R"₇, n, R₈, R₉ et A sont tels que définis précédemment,
   le composé (V) étant un précurseur direct du composé de formule générale (I bis) : 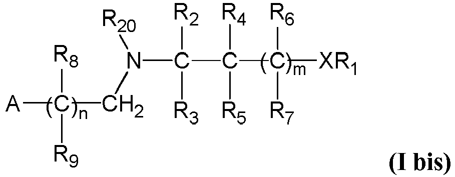
   dans laquelle :
   R₂₀ = H ou R₁₃ tel que défini précédemment,
   X, R₁, m, R₂, R₃, R₄, R₅, R₆, R₇, n, R₈, R₉ et A sont tels que définis précédemment,
   le composé (I bis) étant obtenu à partir du composé (V) selon une des méthodes connues de l'homme de l'art.

Le composé (I bis) forme le radical (I) au sein du peptide C^{α}-amide de l'invention (voir par exemple le composé de formule générale (II)).

Selon un mode de réalisation avantageux du procédé de préparation du composé (I bis) de l'invention, l'étape de mono-*N-*alkylation du composé (IV bis) comporte une réaction d'amination réductrice dudit composé (IV bis) à l'aide d'un aldéhyde de formule générale (VI) : 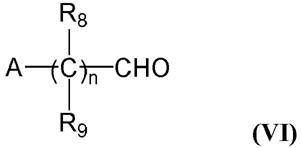 dans laquelle A, n, R₈ et R₉ sont tels que définis précédemment,
afin de former une imine qui est réduite pour former le composé (V) tel que défini ci-dessus.

La réduction pourra par exemple être effectuée à l'aide d'un borohydrure choisi dans le groupe comprenant le cyanoborohydrure de sodium (NaBH₃CN), le triacétoxyborohydrure de sodium (NaBH(OAc)₃) ou le borohydrure de sodium (NaBH₄).

Selon un autre mode de réalisation avantageux du procédé de préparation du composé (I bis) de l'invention, l'étape de mono-*N-*alkylation du composé (IV bis) comporte :
- une étape préalable de sulfonylation de la fonction amine primaire du composé (IV bis), afin d'obtenir le composé de formule (IV ter) : 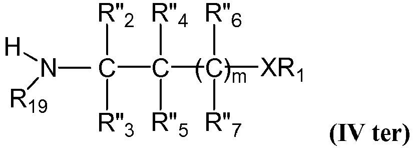
   dans laquelle R₁₉ représente un groupement arylsulfonyle choisi dans le groupe comportant 2-nitrobenzènesulfonyle, 4-nitrobenzènesulfonyle et 2,4-dinitrobenzènesulfonyle,
   m, X, R₁, R"₂, R"₃, R"₄, R"₅, R"₆ et R"₇ sont tels que définis précédemment,
- ladite étape de sulfonylation étant suivie d'une étape de monoalkylation par *N-*substitution nucléophile entre le composé (IV ter) et un composé de formule générale (VII) : 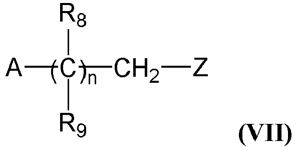
   dans laquelle Z représente un groupe partant choisi dans le groupe comprenant Cl, Br, I ou un sulfonate, ledit sulfonate pouvant par exemple être un « O-mésylate » (-OMs) représenté par MeSO₂-O-, un « O-tosylate » (-OTs) représenté par *p*-Me-C₆H₄-SO₂-O- ou un « O-triflate » (-OTf) représenté par CF₃SO₂-O-,
   A, n, R₈ et R₉ sont tels que définis précédemment,
   pour obtenir le composé de formule (V bis) : 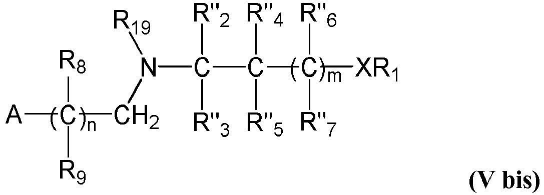
   dans laquelle m, X, R1, R"₂, R"₇ R"₇ R"₇ R"₆, R"₇, A, n, R₈ et R₉ sont tels que définis précédemment,
- ladite étape de monoalkylation étant suivie d'une étape de coupure du groupement sulfonyle R₁₉ du composé (V bis) afin d'obtenir le composé de formule générale (V), le composé (V) étant un précurseur direct du composé de formule générale (I bis), ledit composé (I bis) formant le radical (I) au sein du peptide C^{α}-amide de l'invention.

Selon un deuxième mode de réalisation avantageux de l'invention, le procédé de préparation du composé (I bis) est caractérisé en ce qu'il comporte :
- une étape de mono-*N-*alkylation d'un composé de formule générale (III bis) : 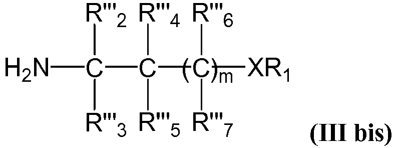
   dans laquelle X, R₁, m, sont tels que définis précédemment,
   un des R'''₂, R'''₃, R'''₄, R'''₅, R'''₆ ou R'''₇ représente un groupe réactif comprenant une fonction chimique réactive éventuellement protégée par un groupe protecteur, ladite fonction étant choisie de préférence dans le groupe comprenant -COOH, -NH₂ ou -OH, et permettant de greffer ledit composé (III bis) sur le support solide tel que mentionné ci-dessus selon une méthode connue de l'homme de l'art pour la SPPS en stratégie Fmoc, ledit groupe protecteur éventuel étant choisi de préférence dans un groupe comprenant tert-butyle, tert-butyloxycarbonyle, phénacyle, benzyle, benzyloxycarbonyle, méthyle, éthyle, trityle et allyle,
   les autres desdits R'''₂, R'''₃, R'''₄, R'''₅, R'''₆ ou R'''₇ qui ne représentent pas ledit groupe réactif ont la même signification que lesdits R₂, R₃, R₄, R₅, R₆ ou R₇ tels que définis précédemment et qui ne représentent pas -B-C-D,
   afin d'obtenir un composé de formule générale (VIII) : 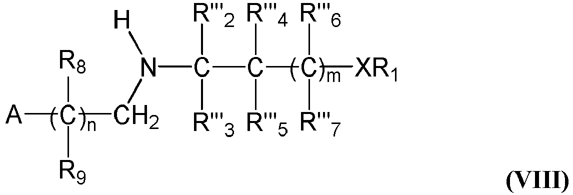
   dans laquelle X, R₁, m, R'''₂, R'''₃, R"₇ R'''₅, R'''₆, R'''₇, n, R₈, R₉ et A sont tels que définis précédemment,
- une étape de *N*-acylation ou de protection de la fonction amine secondaire du composé de formule (VIII) afin d'obtenir un composé de formule générale (VIII bis) : 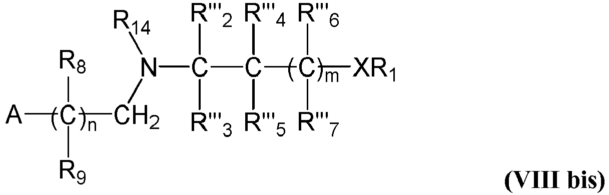 dans laquelle R₁₄ = R₁₃ tel que défini précédemment ou R₁₄ représente un résidu amino acyle protégé de formule R₁₃-Xaa- avec Xaa représentant un résidu d'acide aminé, les éventuelles fonctions réactives de la chaîne latérale du résidu d'acide aminé Xaa étant protégées par des groupes protecteurs habituellement utilisés par l'homme de l'art lors de la SPPS,
- une étape éventuelle de déprotection de la fonction réactive comprise dans l'un desdits R''', pour donner un composé de formule générale (VIII ter) 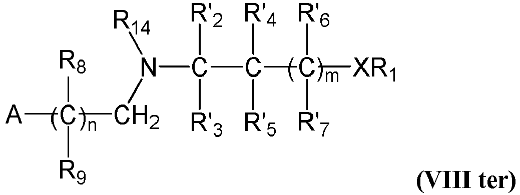
- une étape de greffage du composé (VIII ter) sur un support solide de formule :

   H-E-O-C-D', H-E-NH-C-D' ou Cl-C-D

   dans lequel E, C et D' sont tels que définis précédemment et H, O, NH et Cl correspondant aux symboles chimiques habituellement utilisés, afin d'obtenir le composé de formule (V ter) : 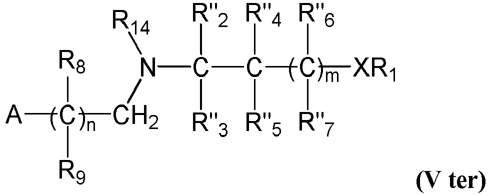 dans laquelle
   X, R₁, m, R"₂, R"₃, R"4, R"₅, R"₆, R"₇ R₁₄, n, R₈, R₉ et A sont tels que définis précédemment,
- dans le cas où R₁₄ = R₁₃, ladite étape de greffage étant suivie d'une étape de coupure du groupement R₁₄ du composé (V ter) afin d'obtenir un composé de formule générale (V) tel que défini précédemment, ledit composé (V) étant un précurseur direct d'un composé de formule (I bis) tel que défini précédemment,
- dans le cas où R₁₄ = R₁₃-Xaa-, ladite étape de greffage étant suivie d'une étape de coupure du groupement R₁₃ faisant partie du groupement R₁₄ du composé (V ter) afin d'obtenir un composé de formule générale (V quater), 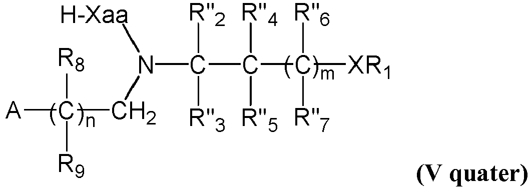
ledit composé (V quater) étant le précurseur d'un peptide C^{α}-amide de l'invention comportant un radical (I), par élongation de la chaîne peptidique par SPPS.

L'invention a encore pour objet un composé caractérisé en ce qu'il présente la formule générale (I bis) : 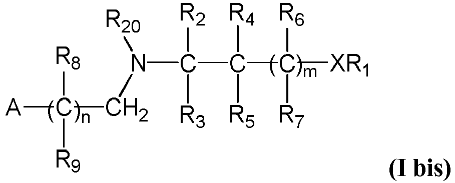 dans laquelle :
X, R₁, m, R₂, R₃, R₄, R₅, R₆, R₇, n, R₈, R₉ et A sont tels que définis précédemment et R₂₀ représente R₁₄,
avec R₁₄ = R₁₃ et R₁₃ représente un groupement protecteur de la fonction amine, et de préférence un groupement Fmoc,
ou R₁₄ représente un résidu amino acyle protégé de formule R₁₃-Xaa- avec Xaa représentant un résidu d'acide aminé provenant d'un acide aminé de formule H-Xaa-OH.

L'invention a encore pour objet un composé caractérisé en ce qu'il présente la formule générale (I bis) : 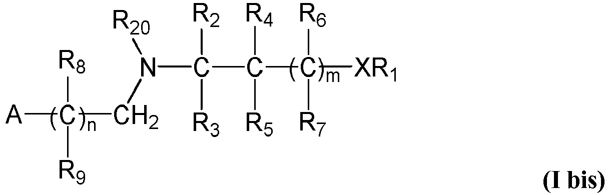 dans laquelle :
X, R₁, m, n, R₈, R₉ et A sont tels que définis précédemment,
un des R₂, R₃, R₄, R₅, R₆ ou R₇ représente le radical -B-C-D dans lequel :
   D représente un atome d'hydrogène ou un support solide convenant pour la synthèse de peptides en phase solide (SPPS),
   C est absent ou représente un bras utilisable pour la SPPS,
   B représente : 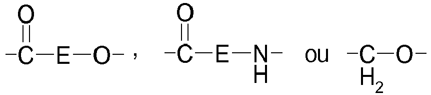
   dans lequel
   E est absent ou représente le groupe -(Xaa)ᵢ- dans lequel :
      i représente un entier allant de 1 à 20, et de préférence de 1 à 5,
      chaque Xaa représente indépendamment les uns des autres un résidu d'acide aminé, et lorsque i est supérieur ou égal à 2, chacun desdits Xaa est relié à son voisin Xaa par une liaison peptidique,
      ou, dans le cas où m =1, et R₄ ou R₅ = -B-C-D, alors le radical divalent B représente de préférence : 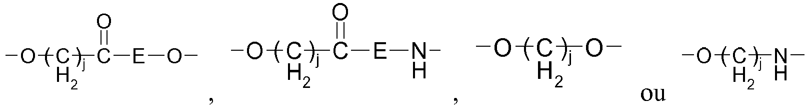
   dans lequel :
   j représente un entier allant de 0 à 10, de préférence de 0 à 3 et E est tel que défini précédemment,
   les autres desdits R₂, R₃, R₄, R₅, R₆ ou R₇ qui ne représentent pas -B-C-D représentent alors indépendamment l'un de l'autre un atome d'hydrogène, un radical alkyle ayant de 1 à 5 atomes de carbone, et de préférence un radical méthyle (-CH₃) ou un radical phényle (-C₆H₅), à la condition qu'au plus deux desdits radicaux R₂, R₃, R₄, R₅, R₆ ou R₇ représentent en même temps un phényle,
R₂₀ représente un hydrogène ou R₁₄,
avec R₁₄ = R₁₃ et R₁₃ représente un groupement protecteur de la fonction amine, et de préférence un groupement Fmoc,
ou R₁₄ représente un résidu amino acyle protégé de formule R₁₃-Xaa- avec Xaa représentant un résidu d'acide aminé provenant d'un acide aminé de formule H-Xaa-OH.

L'invention a encore pour objet un procédé de préparation d'un peptide C^{α}-amide de formule (II) tel que défini ci-dessus par SPPS en stratégie Fmoc, caractérisé en ce qu'il comporte une étape d'élongation par Fmoc SPPS du composé (I bis) tel que défini précédemment, ladite étape d'élongation permettant d'ajouter Peptide1 tel que défini précédemment.

De manière avantageuse selon l'invention l'accrochage du premier acide aminé sur le composé (I bis) par *N*-acylation (à savoir l'ajout d'un acide aminé sur l'azote du composé (I bis)), pourra être effectué de manière très aisée grâce à la structure même du composé (I bis).

En effet l'utilisation du composé de l'invention (I bis) est extrêmement avantageuse, comparée par exemple au composé (h) de l'art antérieur défini à la figure 5-1, puisque c'est la structure originale dudit composé (I bis) qui permet de faciliter cette étape de N-acylation de l'amine secondaire.

La présente invention a encore pour objet un procédé de préparation d'un peptide C^{α}-thioester de formule générale (IX) :

Peptide1 - S - R' **(IX)**

dans laquelle :
R'- représente un radical provenant d'un thiol de formule R'-SH, et est un groupement alkyle, aryle, arylalkyle, cycloalkyle, hétérocycloalkyle, hétéroaryle, pouvant comporter un ou plusieurs substituants choisis parmi halogène, carboxyle, sulfonate, ammonium, alcool, éther, alkyle, cycloalkyle, hétérocycloalkyle, aryle, hétéroaryle, alcoxy, halogénoalkyle, arylalkyle, hétéroarylalkyle, arylhétérocycloalkyle,
Peptide1 est tel que défini précédemment,
caractérisé en ce qu'il comprend une réaction de thio-estérification entre :
   - le peptide C^{α}-amide de formule (II) tel que défini précédemment et dans laquelle R₁ = H et,
   - un thiol de formule R'-SH,
   afin d'obtenir :
   - le peptide C^{α}-thioester de formule (IX) tel que défini ci-dessus et,
   - le composé de formule (I ter) : 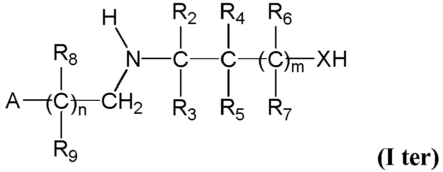
dans laquelle : X, m, R₂, R₃, R₄, R₅, R₆, R₇, n, R₈, R₉ et A sont tels que définis précédemment.

Le composé (I ter) correspond au composé de formule générale (I bis) dans laquelle R₁ et R₂₀ représente chacun un atome d'hydrogène.

Le radical R'- provenant du thiol de formule R'-SH, peut représenter n'importe quel radical permettant de former un thiol (R'-SH) lorsqu'il est lié à la fonction SH du thiol. 1.

Le radical R'- pourra par exemple représenter un groupement alkyle, aryle, arylalkyle, cycloalkyle, hétérocycloalkyle, hétéroaryle tel que défini ci-dessus, chacun desdits groupements pouvant en outre comporter un ou plusieurs substituants classiques choisis parmi : halogène, carboxyle, sulfonate, ammonium, alcool, éther, alkyle, cycloalkyle, hétérocycloalkyle, aryle, hétéroaryle, alcoxy, halogénoalkyle, arylalkyle, hétéroarylalkyle, arylhétérocycloalkyle.

A titre d'exemple de radical alkyle on pourra citer un de ceux choisis dans le groupe comprenant éthyle, 2-hydroxyéthyle, 3-hydroxypropyle, NaSO₃CH₂CH₂-, HOOC-CH₂CH₂-, HOOC-CH₂- ou CH₃CH₂COOCH₂CH₂-.

A titre d'exemple de radical aryle ou arylalkyle on pourra citer un de ceux choisis dans le groupe comprenant benzyle (C₆H₅-CH₂-), 4-méthoxybenzyle (p-MeO-C₆H₅-CH₂-), 2,4,6-triméthoxybenzyle, 4-méthylbenzyle, phényle (C₆H₅-), p-(HOOC-CH₂)-C₆H₄-, p-CF₃-C₆H₄-, p-F-C₆H₄-, p-Cl-C₆H₄-, p-Br-C₆H₄-, p-I-C₆H₄-, p-NO₂-C₆H₄-, p-Me-C₆H₄- ou p-HO-CH₂-C₆H₄-.

La réaction entre le peptide C^{α}-amide de formule (II) et le thiol R'-SH pourra être effectuée en présence d'un excès de thiol dans de l'acide trifluoroacétique (TFA), de l'acide acétique, de l'acide formique ou dans un tampon aqueux, éventuellement additionné d'un co-solvant organique, d'urée ou de chlorure de guanidinium, pour assurer la solubilisation du peptide C^{α}-amide de formule (II) ou du thiol R'-SH.

Le pH du tampon aqueux pourra être de 0 à 8, et de préférence de 1 à 4.

A titre d'exemple de co-solvant organique on pourra citer un de ceux choisis dans le groupe comprenant l'acétonitrile (MeCN), le méthanol (MeOH), l'isopropanol (iPrOH), le diméthylformamide (DMF) ou la N-méthyl-2-pyrrolidone (NMP).

De manière préférée, la réaction pourra être effectuée dans un mélange eau/MeCN 7:3, en présence de 0,1% v/v TFA et de 50 équivalents d'un thiol R'SH.

Dans un mode particulier de préparation d'un peptide C^{α}-thioester de formule (IX), D représente un support solide convenant pour la SPPS en stratégie Fmoc et C est absent ou représente un bras utilisable pour la SPPS en stratégie Fmoc et de préférence un bras stable au TFA tel que le bras PAM ou MBHA. Dans ce mode particulier, l'obtention du peptide C^{α}-thioester de formule (IX) est effectuée à l'aide, dans un premier temps, d'un traitement au TFA de la peptidyle résine pour couper les groupements protecteurs des chaînes latérales de Peptide1 utilisés lors de l'étape d'élongation du peptide 1 par Fmoc SPPS, sans le décrocher du support solide. Puis, dans un deuxième temps, on effectue la thio-estérification du peptide déprotégé supporté obtenu après le traitement au TFA à l'aide du thiol R'-SH. Ceci a également pour effet de décrocher le peptide C^{α}-thioester de formule (IX) du support solide.

De façon avantageuse selon l'invention, le peptide C^{α}-amide tel que défini ci-dessus pourra être utilisé pour préparer un peptide C^{α}-thioester. L'invention a donc aussi pour objet l'utilisation d'un peptide C^{α}-amide tel que défini ci-dessus pour préparer un peptide C^{α}-thioester.

L'invention concerne encore un procédé de préparation d'un peptide de formule générale (X) :

Peptide1-Yaa-Peptide2-R₁₅ **(X)**

dans laquelle :
Peptide1 est tel que défini précédemment,
Yaa est un résidu d'acide aminé provenant d'un acide aminé de formule H-Yaa-OH choisi dans le groupe comprenant une cystéine, une homocystéine, une β-mercapto-valine, une β-mercapto-leucine, une β-mercapto-isoleucine, une β-mercapto-phénylalanine, une β-mercapto-lysine, une β-mercapto-proline, une γ-mercapto-valine, une γ-mercapto-isoleucine, une γ-mercapto-leucine, une γ-mercapto-lysine, une γ-mercapto-proline, ou un acide aminé substitué sur son atome d'azote N^{α} par un groupement contenant une fonction β- ou γ-amino thiol ledit groupement étant choisi dans le groupe comprenant: 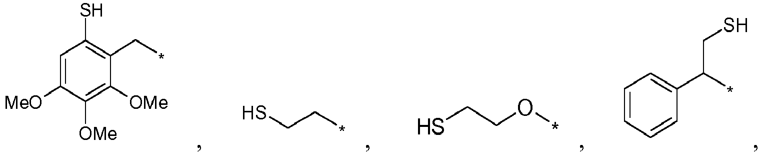 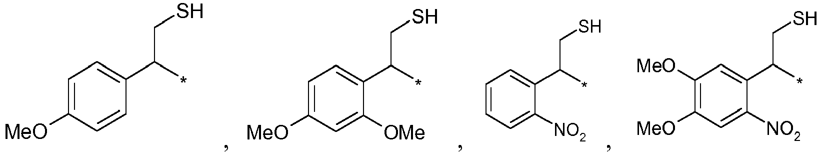 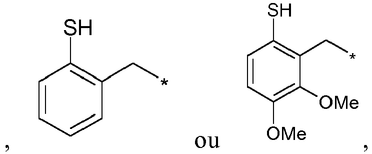
Peptide2= (Xaa)₁, avec 1 = un entier allant de 1 à 60, de préférence de 4 à 40, Xaa représente indépendamment les uns des autres un résidu d'acide aminé provenant d'un acide aminé de formule H-Xaa-OH, et lorsque 1 est supérieur ou égal à 2, chacun desdits Xaa étant relié à son voisin Xaa par une liaison peptidique,
R₁₅ représente -OH, -NH₂ ou un radical de formule générale (I) tel que défini ci-dessus, dans laquelle X= S et R₁ est un groupe protecteur du soufre qui est stable à un traitement au TFA et stable dans des conditions de NCL,
caractérisé en ce que l'on fait réagir par une réaction de NCL :
un peptide C^{α}-amide de formule générale (II) tel que défini ci-dessus, dans laquelle R₁ = H ou un groupement labile dans des conditions de ligation chimique native NCL,
avec un peptide possédant un résidu β- ou γ-amino thiol N terminal de formule générale (XI) :

   H-Yaa-Peptide2-R₁₅ **(XI)**
dans laquelle Yaa, R₁₅ et Peptide2 sont tels que définis ci-dessus, afin d'obtenir
   - le peptide de formule générale (X) tel que défini ci-dessus et
   - le composé de formule (I bis) tel que défini ci-dessus.

De façon avantageuse selon l'invention, les peptides C^{α}-amides présentent des propriétés de crypto-thioesters et à cet égard peuvent donc être utilisés directement dans une réaction de ligation chimique native NCL, sans être convertis préalablement en peptides C^{α}-thioesters. L'invention a donc aussi pour objet l'utilisation d'un peptide C^{α}-amide tel que défini ci-dessus dans une réaction de ligation native NCL (« Native Chemical Ligation ») pour préparer un peptide ou une protéine, en particulier d'intérêt thérapeutique.

La présente invention a encore pour objet un procédé de préparation d'un peptide de formule générale (XII) :

Peptide1-Yaa₂-Peptide2-...-Yaa_{q-1}-Peptide(q-1)-Yaa_{q}-Peptide(q)-R₁₅ **(XII)**

dans laquelle :
R₁₅ est tel que défini précédemment,
q est un entier allant de 3 à 10,
chaque Yaa (Yaa₂, ..., Yaa_{q-1} et Yaaq) représente indépendamment les uns des autres un résidu d'acide aminé tel que défini précédemment,
chaque Peptide de la formule (XII) ci-dessus (Peptide2 ... Peptide(q-1) et Peptide(q)), excepté le Peptide1, représente indépendamment les uns des autres (Xaa)₁ tel que défini précédemment à propos de Peptide2,
caractérisé en ce que l'on fait réagir par une première réaction de NCL :
un peptide de formule (II) tel que défini précédemment dans laquelle R₁ = H ou
un groupement labile dans des conditions de ligation chimique native NCL,
avec un peptide de formule générale (XI bis) :

   H-Yaa₂-Peptide2-R₁₆ **(XI bis)**
dans laquelle :
Yaa₂ est égal à Yaa tel que défini précédemment, et Peptide2 est tel que défini précédemment, R₁₆ représente un radical de formule (I) dans laquelle R₁ représente un groupement protecteur stable dans des conditions de NCL,
afin d'obtenir un peptide de formule générale (XIII) :

   Peptide1-Yaa₂-Peptide2-R₁₆ **(XIII)**
dans laquelle :
Yaa₂, Peptide1, Peptide2 et R₁₆ sont tels que définis précédemment,
et un composé de formule (I ter) tel que défini précédemment.

Le peptide de formule générale (XIII) présentant le radical R₁₆ tel que défini précédemment est ensuite soumis à une déprotection dans les conditions connues de l'homme de l'art afin d'obtenir le peptide de formule générale (XIII bis) :

Peptide1-Yaa₂-Peptide2-R₁₇ **(XIII bis)**

dans laquelle :
R₁₇ représente un radical de formule (I) dans laquelle R₁ représente un hydrogène.

Le composé (XIII bis) ainsi obtenu est :
- soit converti au préalable en peptide thioester puis mis à réagir avec un peptide de formule générale (XI ter) :

   H-Yaa₃-Peptide3-R₁₆ **(XI ter)**,

   ou
- soit directement mis à réagir avec le peptide de formule (XI ter), en exploitant les propriétés de crypto-thioester du composé (XIII bis).

Cet assemblage successif est continué de façon itérative, en répétant (q-3) fois les étapes successives de NCL et de déprotection telles que décrites ci-dessus.

Le composé final de formule générale (XII) est obtenu par un dernier assemblage permettant de rajouter le Peptide(q).

Ce dernier assemblage s'effectue :
- soit en deux étapes, à savoir conversion préalable en thioester puis réaction avec un peptide de formule générale (XI quater) :

   H-Yaa_{q}-Peptide(q)-R₁₅ **(XI quater),**

   ou
- soit par réaction directe avec ledit peptide de formule (XI quater).

Lorsque q est un entier égal à 3, alors le peptide de formule (XII) est représenté par la formule (XII) suivante :

Peptide1-Yaa₂-Peptide2-Yaa₃-Peptide₃-R₁₅ **(XII).**

Le composé de formule (XI bis) est un peptide C^{α}-amide qui ne présente pas des propriétés de crypto-thioester du fait du masquage de sa fonction thiol par un radical R₁ représentant un groupe protecteur du soufre stable à un traitement au TFA et stable dans des conditions de NCL.

Un groupement R₁ préféré du radical (I) présent dans ledit composé (XI bis) est plus particulièrement un groupement choisi dans le groupe comprenant acétamidométhyle (Acm), triméthylacétamidométhyle (Tacm), benzamidométhyle (Bam), allyloxycarbonylaminométhyle (Allocam), phtalimidométhyle (Pim), 2-(triméthylsilyl)éthyle, t-butyle (tBu), 2,4,6-triméthylbenzyle, quinolinylméthyle (Qm), diphényl-4-pyridylméthyle, 1-adamantyle, benzyloxyméthyle (BOM), 2-tetrahydropyranyle (Thp) et benzylthiométhyle.

Selon un mode de réalisation préféré du procédé de préparation du peptide de formule générale (XII), le Peptide1 est préalablement greffé sur un support solide hydrocompatible, comme par exemple un support choisi dans un groupe comprenant PEGA®, CHEMMATRIX™, l'agarose, le sépharose et des microparticules de verre à porosité contrôlée (CPG, controlled pore glass). Plus particulièrement le Peptide1 est greffé sur un support solide par un lien pouvant être coupé après les (q-1) étapes de ligation chimique native NCL, ledit lien étant de préférence un bras N-terminal tel que décrit dans le document WO 2011/058188. D'autres réactions de ligations (nécessitant éventuellement d'autres types de précurseurs) pourront également être combinées avec la NCL dans une même stratégie de ligations multiples sur support solide de N vers C.

L'invention sera mieux comprise à la lumière des exemples non limitatifs et purement illustratifs suivants. Les figures 6 à 26 permettent d'illustrer lesdits exemples ci-dessous.
Les figures 1 à 5, concernant l'art antérieur, ont été commentées ci-dessus dans l'introduction de la présente demande.
La figure 1 représente les étapes de la Synthèse Peptidique sur Phase Solide (SPPS).
La figure 2 représente les groupements protecteurs des amines (Boc et Fmoc) utilisés en SPPS.
La figure 3 représente une réaction de ligation chimique native « NCL » entre un peptide C^{α}-thioster (composé (a)) et un peptide portant une cystéine N-terminale (composé (b)).
La figure 4 représente l'utilisation de peptides C^{α}-*N*-alkyl(ou aryl)-*N*-(β-mercaptoalkyl)-amides (e) pour :
   - la synthèse de peptides (a) (par transfert d'acyle de *N* vers *S*) (Fig. 4-1 et 4-2),
   - l'utilisation directe en NCL (Fig. 4-3).
La figure 5-1 représente un schéma de principe de synthèse de peptides C^{α}-β-mercapto-amides de type (e), détaillant l'étape difficile de *N*-acylation de l'amine secondaire.
La figure 5-2 est une comparaison entre l'acylation d'une amine secondaire portant un groupement 2-hydroxybenzyle (j) et celle d'une amine secondaire portant un groupement 2-méthoxybenzyle (m), et illustre plus particulièrement le principe de l'assistance par un groupement 2-hydroxybenzyle de l'acylation d'une amine secondaire.
La figure 6 est un schéma de synthèse illustrant la préparation de six composés de l'invention répondant à la formule générale (I bis) (composés **3a, 3b, 3c, 3'a, 3'b** et **3'c**).
La figure 7 est un schéma de synthèse illustrant la préparation d'un peptide C^{α}-amide de formule générale (II) (composés **5** et **5'**) à partir d'un composé de formule générale (I bis) (composé **3a**).
La figure 8 représente le chromatogramme d'une analyse par HPLC/MS du peptide **2'** représenté à la figure 6.
Les figures 9, 10 et 11 représentent les chromatogrammes d'analyses par HPLC/MS respectifs des peptides **3'a, 3'b** et **3'c** représentés à la figure 6.
Les figures 12 et 13 représentent les chromatogrammes d'analyses par HPLC/MS respectifs des peptides **4'a** et **4'b** représentés à la figure 7.
La figure 14 représente le chromatogramme d'une analyse par HPLC/MS du peptide **5'** représenté à la figure 7.
La figure 15 représente l'utilisation d'un peptide C^{α}-amide de formule générale (II) (composé **5'**) pour obtenir un peptide C^{α}-thioester (composé **6**).
La figure 16 représente l'utilisation d'un peptide C^{α}-amide de formule générale (II) (composé **5'**) dans une réaction de NCL pour obtenir un peptide modèle (composé **9**) (réactivité de type crypto-thioester).
La figure 17 est un schéma de synthèse illustrant la préparation de trois composés de l'invention répondant à la formule générale (I bis) (composés **12, 13** et **13'**).
La figure 18 représente le chromatogramme d'une analyse par HPLC/MS du composé **13'** représenté à la figure 17.
La figure 19 est un schéma de synthèse illustrant la préparation d'un peptide C^{α}-amide de formule générale (II) (composés **14** et **14'**) à partir d'un composé de formule générale (I bis) (composé **12**).
La figure 20 représente le chromatogramme d'une analyse par HPLC/MS des peptides **14'** et **14"** représentés à la figure 19.
La figure 21 est un schéma de synthèse illustrant la préparation d'un peptide C^{α}-amide de formule générale (II) (composés **15** et **15'**) à partir d'un composé de formule générale (I bis) (composé **13**).
La figure 22 représente le chromatogramme d'une analyse par HPLC/MS du peptide **15'** représenté à la figure 21.
La figure 23 représente l'utilisation des peptides **14'/14"** dans une réaction de ligation chimique native NCL pour donner un peptide **17,** suivi d'un repliement oxydatif *one-pot* afin d'obtenir un peptide **19.**
La figure 24 représente le chromatogramme d'une analyse par HPLC/MS du peptide **16** représenté à la figure 23.
La figure 25 représente le chromatogramme d'une analyse par HPLC/MS du peptide **17** représenté à la figure 23.
La figure 26 représente le chromatogramme d'une analyse par HPLC/MS du peptide **19** représenté à la figure 23.

### EXEMPLES

Dans les exemples 1 et 2 décrits ci-dessous, dans le radical de formule (I) ou dans le composé (I bis):
X représente un atome de soufre,
R₁ représente un atome d'hydrogène ou un groupe trityle (Trt),
R₂ représente un groupe -B-C-D dans lequel :
   B représente -CO-Leu-Tyr-Arg-Ala-Gly-O-, C est absent et D représente un atome d'hydrogène, ou
   B représente -CO-Leu-Tyr(t-Bu)-Arg(Pbf)-Ala-Gly-O-, C représente un bras de Wang (-CH₂-C₆H₄-O-CH₂-) et D représente un support solide tel qu'une résine de type polystyrène,
R₃, R₄ et R₅ représentent chacun un atome d'hydrogène
m est égal à n qui est égal à 0,

A représentant un groupe aryle de formule générale : 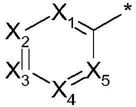
dans laquelle X₁ représente C-OH, chacun des X₂, X₃ et X₅ représente CH et X₄ représente CH, C-OCH₃ ou C-NO₂,
et, si il s'agit du composé (I bis), R₂₀ représente un atome d'hydrogène.

Lorsque D représente un atome d'hydrogène cela signifie que le radical (I) n'est pas accroché sur un support solide. Dans ce cas R₁ représente H et R₂ représente un groupe -B-C-D dans lequel B représente -CO-Leu-Tyr-Arg-Ala-Gly-O-, C est absent et D représente un atome d'hydrogène.

Lorsque D représente un support solide cela signifie que le radical (I) est accroché sur ledit support solide (ledit support solide faisant partie intégrante de la définition du radical (I)). Dans ce cas R₁ représente le trityle (Trt) et R₂ représente un groupe -B-C-D dans lequel B représente -CO-Leu-Tyr(t-Bu)-Arg(Pbf)-Ala-Gly-O-, C représente un bras de Wang (-CH₂-C₆H₄-O-CH₂-) et D représente un support solide tel qu'une résine de type polystyrène.

Les produits obtenus ont été analysés par HPLC sur Colonne : nucleosil C18 300A 5µm, 4,6x250 mm, gradient : 5-50% MeCN/H₂O + 0,1% TFA en 30 min, débit : 1 ml/min, suivie d'une détection UV (λ=276 nm ou 360 nm) puis d'une spectrométrie de masse MALDI-TOF.

### Exemple 1 :

### Préparation d'un peptide C^{α}-amide de formule générale (II)

### 1) Synthèse d'un composé de formule générale (I bis)

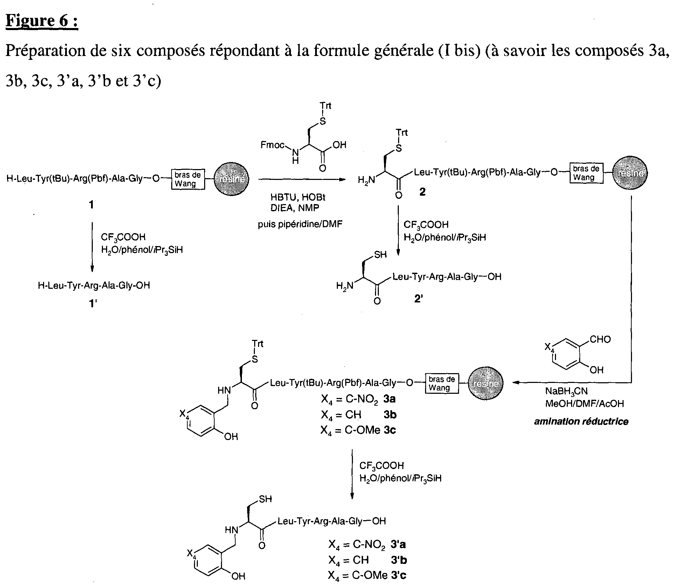

La synthèse d'un composé de formule générale (I bis) est illustrée à la figure 6. Les composés **3a, 3b, 3c** et **3'a, 3'b** et **3'c** répondent à la formule générale (I bis) dans laquelle :
→ pour les composés **3a, 3b et 3c,** X représente S, R₁ représente Trt, R₃, R₄ et R₅ représentent H, m est égal à n qui est égal à 0, R₂₀ représente un atome d'hydrogène, R₂ représente -CO-Leu-Tyr(t-Bu)-Arg(Pbf)-Ala-Gly-O-bras de Wang-Résine, et A représente respectivement : 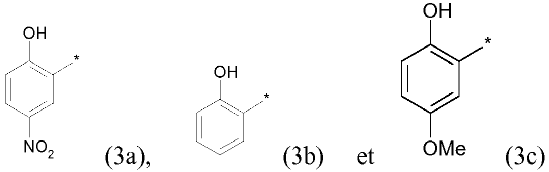
→ pour les composés **3'a, 3'b et 3'c,** X représente S, R₁ représente H, R₃, R₄ et R₅ représentent H, m est égal à n qui est égal à 0, R₂₀ représente un atome d'hydrogène, R₂ représente -CO-Leu-Tyr-Arg-Ala-Gly-OH, et A représente respectivement :

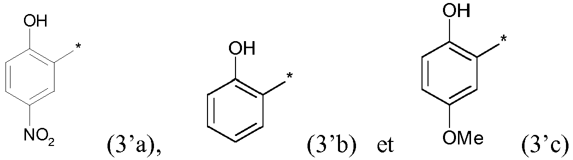

### • Synthèse de la peptidyle résine 2

Le schéma de synthèse est illustré à la figure 6.

Le composé 1 répond à la formule H-E-O-C-D' telle que définie ci-dessus dans laquelle :
E représente Leu-Tyr(t-Bu)-Arg(Pbf)-Ala-Gly,
C représente un bras de Wang et D' représente une résine de type polystyrène.

Le composé 2 répond à la formule générale (IV bis) telle que définie ci-dessus : 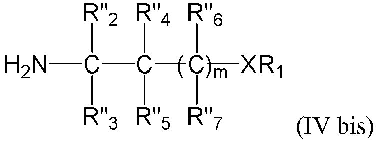 dans laquelle
X représente S, R₁ représente Trt, R"₃, R"₄, R"₅, R"₆ et R"₇ représentent H, m est égal à 0 et, R"₂ représente :
   -CO-Leu-Tyr(t-Bu)-Arg(Pbf)-Ala-Gly-O-bras de Wang-Résine

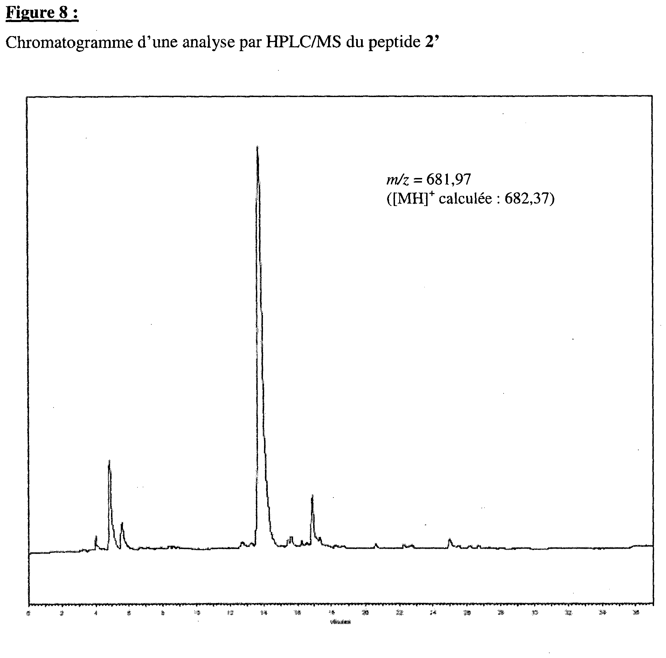

La peptidyle résine 2 de formule :
H-Cys(Trt)-Leu-Tyr(t-Bu)-Arg(Pbf)-Ala-Gly-O-bras de Wang-Résine a été synthétisée dans des conditions standard (synthétiseur ABI 433, programme Fastmoc). Une aliquote de résine est traitée pendant 2h par CF₃COOH/phénol/H₂O/tri-isopropylsilane 87,5:5:5:2,5 pour décrocher et déprotéger le peptide **2** afin d'obtenir le peptide **2',** qui est précipité par dilution dans un mélange éther de pétrole/Et₂O 1 :1, lavé (Et₂O), séché puis analysé par HPLC et spectrométrie de masse (voir figure 8).

Peptide **2'** (voir figure 8):
Formule brute : C₂₉H₄₇N₉O₈S ;
HPLC : t_{R} = 14,41 min (C18, gradient : 5-50% MeCN/H₂O + 0,1%TFA en 30 min) ;
Détection UV (λ=276 nm) ;
MS (MALDI-TOF) : *m*/*z* observée = 682,0 ([MH]⁺ calculée pour C₂₉H₄₇N₉O₈S = 682,3).

### • Aminations réductrices

Le schéma de synthèse est illustré à la figure 6.

0,1 mmol de la peptidyle résine **2** de formule :
H-Cys(Trt)-Leu-Tyr(t-Bu)-Arg(Pbf)-Ala-Gly-O-bras de Wang-Résine
est solvatée dans le mélange diméthylformamide /méthanol/acide acétique (9 :9 :2).

10 équivalents d'aldéhyde (de formule générale VII) tel que représenté à la figure 6 sont ensuite dissouts dans 4 mL de diméthylformamide /méthanol (1 :1) et sont ajoutés à la peptidyle résine **2.** La suspension ainsi obtenue est agitée pendant 45 minutes. La résine est ensuite lavée avec le mélange diméthylformamide /méthanol (1 :1).

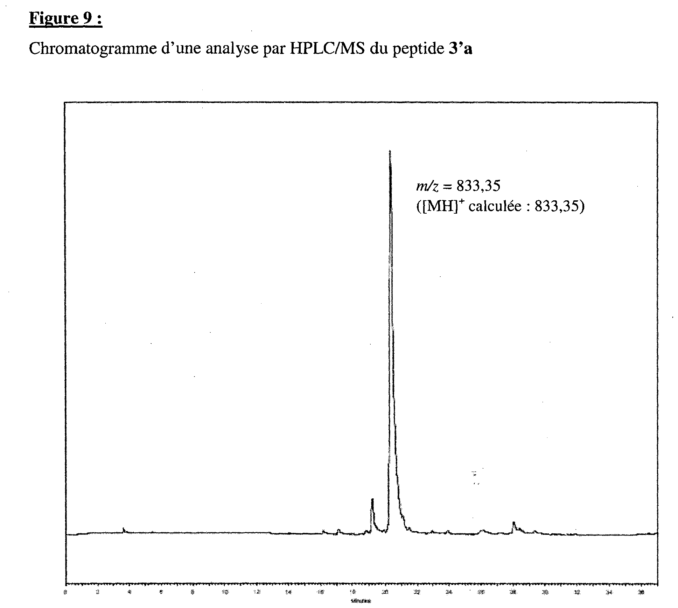

20 équivalents de NaBH₃CN sont dissouts dans 4 mL de diméthylformamide /méthanol/acide acétique (9 :9 :2) puis ajoutés à la résine. La suspension ainsi obtenue est agitée pendant 30 minutes. La résine est lavée avec le mélange diméthylformamide /méthanol/acide acétique (9 :9 :2). Une aliquote de résine est traitée pendant 2h par TFA/PhOH/H₂O/*i*-Pr₃SiH 87,5:5:5:2,5 pour décrocher et déprotéger le peptide **3a, 3b** ou **3c** afin d'obtenir respectivement le peptide **3'a, 3'b ou 3'c,** qui est précipité par dilution dans un mélange éther de pétrole/Et₂O 1 : 1, lavé (Et₂O), séché puis analysé par HPLC et spectrométrie de masse (voir figures 9, 10 et 11).

Peptide **3'a** (figure 9):
Formule brute : C₃₆H₅₂N₁₀O₁₁S ;
HPLC : t_{R} = 20,44 min (C18, gradient : 5-50% MeCN/H₂O + 0,1%TFA en 30 min) ;
Détection UV (λ= 360 nm) ;
MS (MALDI-TOF) : *m*/*z* observée = 833,3 ([MH]⁺ calculée pour C₃₆H₅₂N₁₀O₁₁S = 833,4).

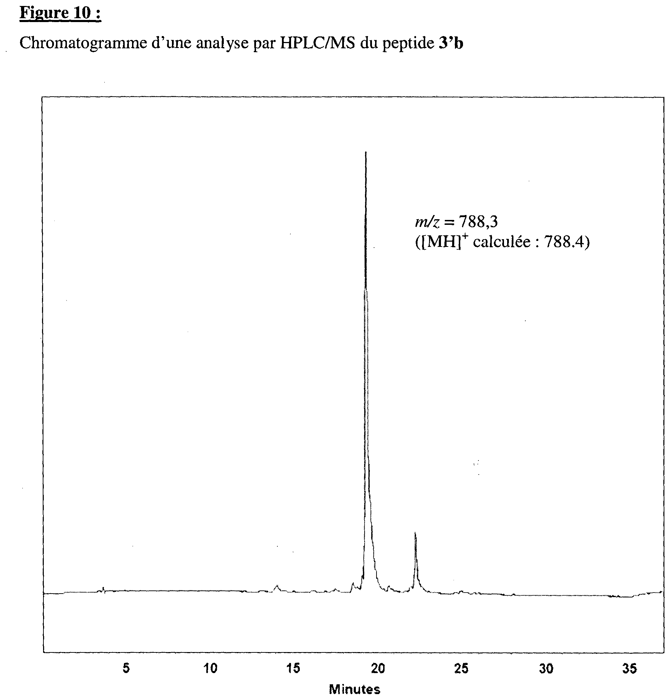

Peptide **3'b** (figure 10) :
Formule brute : C₃₆H₅₃N₉O₉S ;
HPLC : t_{R} = 19,17 min (gradient : 5-50% MeCN/H₂O + 0,1%TFA en 30 min) ;
Détection UV (λ= 276 nm) ;
MS (MALDI-TOF) : *m*/*z* obtenue = 788,3 ([MH]⁺ calculée pour C₃₆H₅₃N₉O₉S = 788,4).

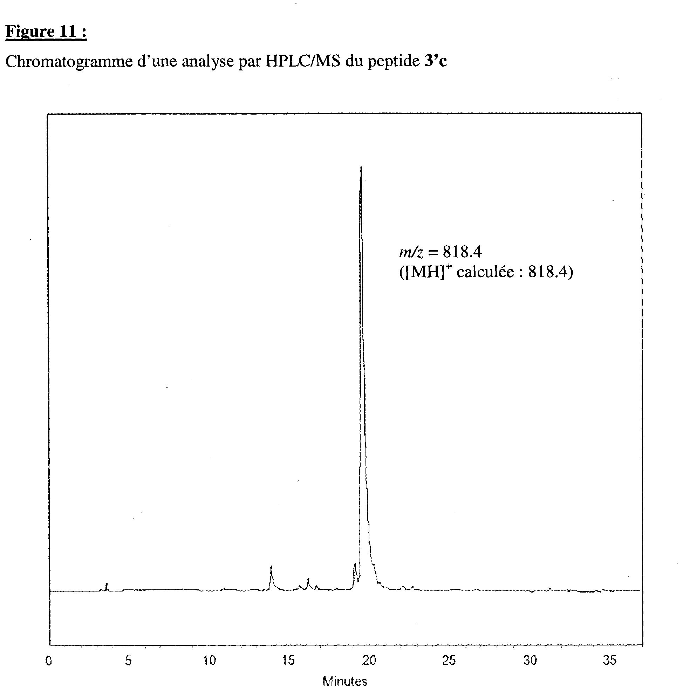

Peptide **3'c** (figure 11) :
Formule brute : C₃₇H₅₅N₉O₁₀S ;
HPLC : t_{R} = 19,59 min (C18, gradient : 5-50% MeCN/H₂O + 0,1%TFA en 30 min) ;
Détection UV (λ= 276 nm) ;
MS (MALDI-TOF) : *m*/*z* obtenue = 818,4 ([MH]⁺ calculée pour C₃₇H₅₅N₉O₁₀S = 818,4).

### 2) Synthèse d'un peptide C^{α}-amide de formule générale (II)

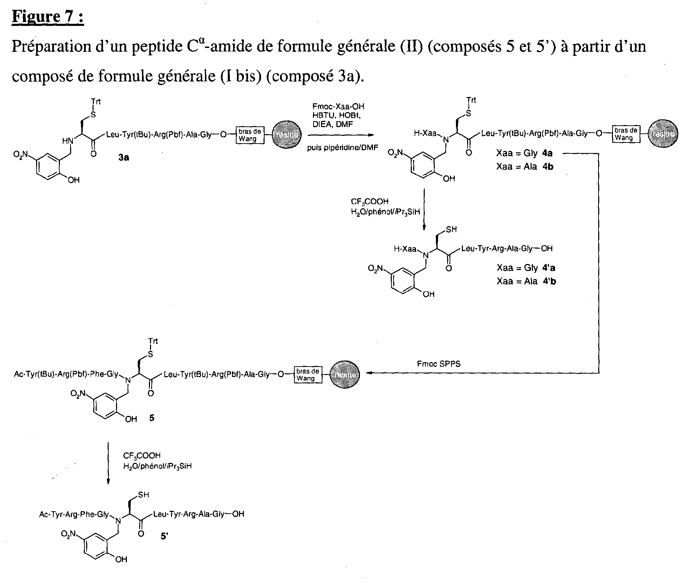

La synthèse d'un peptide C^{α}-amide de formule générale (II) est illustrée à la figure 7. Les composés **5** et **5'** répondent à la formule générale (II) dans laquelle :
→ pour le composé **5,** X représente S, R₁ représente Trt, R₃, R₄ et R₅ représentent H, Peptide 1 représente Ac-Tyr(tBu)-Arg(Pbf)-Phe-Gly, m est égal à n qui est égal à 0, R₂ représente -CO-Leu-Tyr(t-Bu)-Arg(Pbf)-Ala-Gly-O-bras de Wang-Résine, et A représente 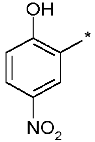
→ pour le composé **5'**, X représente S, R₁ représente H, R₃, R₄ et R₅ représentent H, Peptide 1 représente Ac-Tyr-Arg-Phe-Gly-, m est égal à n qui est égal à 0, R₂ représente -CO-Leu-Tyr-Arg-Ala-Gly-OH, et A représente :

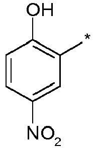

### • Couplage de la Fmoc-glycine sur la N-alkyl cystéine supportée 3a

Le couplage de la Fmoc-glycine sur la peptidyl résine **3a** est illustré à la figure 7.

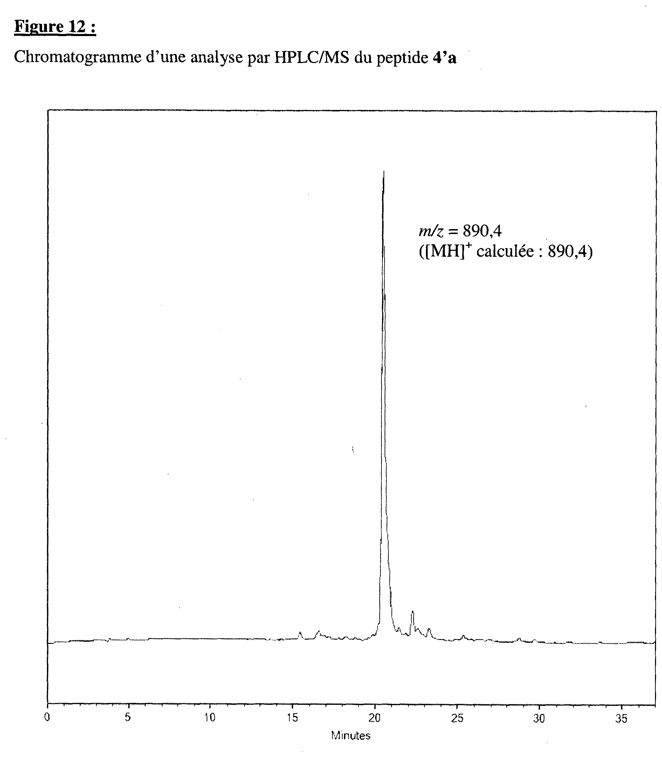

Le couplage de la Fmoc-glycine sur la peptidyl résine **3a** est réalisée dans des conditions standard (ABI 433, programme Fastmoc). La peptidyl résine **3a** est solvatée dans du DMF. Puis 10 équivalents d'acide aminé protégé (Fmoc-Gly-OH tel que représenté sur la figure 7) sont activés par 10 équivalents de HBTU (*O-*benzotriazole-*N*,*N*,*N'*,*N'*-tetramethyl-uronium-hexafluorophosphate) en présence de 10 équivalents de HOBt (1-hydroxybenzotriazole) et 20 équivalents de DIEA (di-isopropylamine) puis ajoutés à la résine, qui est ensuite agitée pendant 30 minutes à température ambiante. La résine est rincée avec DMF et CH₂Cl₂ puis le groupement Fmoc est coupé à l'aide de pipéridine (20% dans la NMP), trois traitements successifs de cinq minutes chacun. La résine est rincée avec du DMF et du CH₂Cl₂. Une aliquote de résine **4a** ainsi obtenue est traitée pendant 2h par TFA/PhOH/H₂O/*i-*Pr₃SiH 87,5:5:5:2,5 pour décrocher et déprotéger le peptide **4a** et obtenir le peptide **4'a**, qui est analysé par HPLC et spectrométrie de masse (figure 12).

Peptide **4'a** (figure 12):
Formule moléculaire : C₃₈H₅₅N₁₁O₁₂S;
HPLC : t_{R} = 20,53 min (C18, gradient : 5-50% MeCN/H₂O + 0,1%TFA en 30 min) ;
Détection UV (λ= 276 nm) ;
MS (MALDI-TOF) : *m*/*z* obtenue = 890,4 ([MH]⁺ calculée pour C₃₈H₅₅N₁₁O₁₂S = 890,4).

### • Couplage de la Fmoc-alanine sur la N-alkyl cystéine supportée 3a

Le couplage de la Fmoc-alanine sur la peptidyle résine **3a** est illustré à la figure 7.

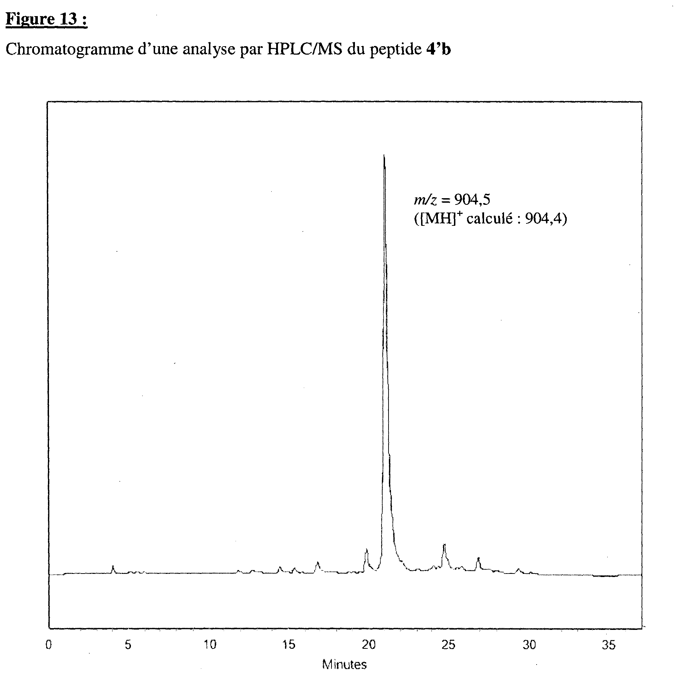

La peptidyle résine **3a** est solvatée dans du DMF. Puis 10 équivalents de Fmoc-alanine protégée (Fmoc-Ala-OH tel que représenté sur la figure 7) sont activés par 10 équivalents de HBTU en présence de 10 équivalents de HOBt et 20 équivalents de DIEA dans le DMF et de la peptidyle résine **3a** pendant 30 minutes à température ambiante. Cette étape est réalisée deux fois. La résine est rincée avec DMF et DCM puis l'amine est déprotégée avec de la pipéridine (20% dans la NMP), à trois reprises pendant cinq minutes. La résine est rincée avec du DMF et du DCM. Une aliquote de résine **4b** ainsi obtenue est traitée pendant 2h par TFA/PhOH/H₂O/*i*-Pr₃SiH 87,5:5:5:2,5 pour décrocher et déprotéger le peptide **4b** et obtenir le peptide **4'b**, qui est analysé par HPLC et spectrométrie de masse (figure 13).

Peptide **4'b** (figure 13) :
Formule moléculaire : C₃₉H₅₇N₁₁O₁₂S ;
HPLC : t_{R} = 21,50 min (gradient : 5-50% MeCN/H₂O + 0,1%TFA en 30 min détection DAD) ;
Détection UV (λ= 276 nm) ;
MS (MALDI-TOF) : *m*/*z* obtenue = 904,5 ([MH]⁺ calculée pour C₃₉H₅₇N₁₁O₁₂S = 904,4);

### • Elongation du peptide 5' à partir du composé 4a obtenu

L'élongation du peptide **5'** est représenté à la figure 7.

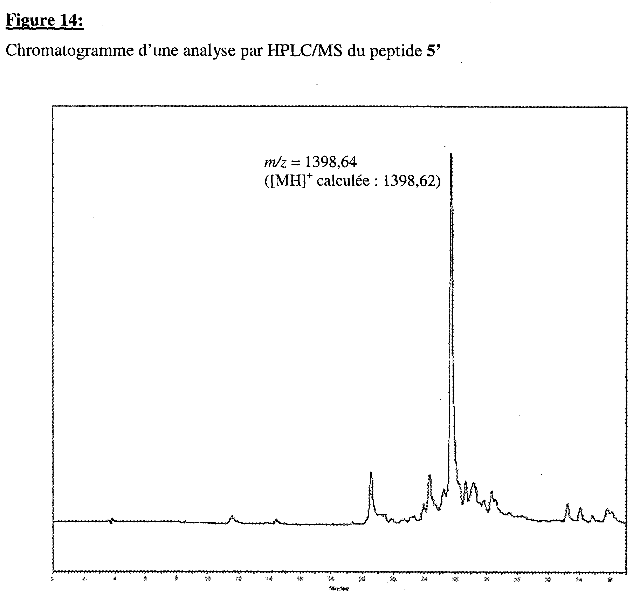

La suite de l'élongation du peptide est réalisée dans des conditions standard (ABI 433, programme Fastmoc) avec la peptidyle résine **4a.** Une aliquote de résine **5** est traitée pendant 2h par TFA/PhOH/H₂O/*i*-Pr₃SiH 87,5:5:5:2,5 pour obtenir le peptide **5'** qui est précipité par dilution dans un mélange éther de pétrole/Et₂O 1 :1, lavé (Et₂O), séché puis analysé par HPLC et spectrométrie de masse (figure 14).

Peptide **5'** (figure 14) :
Formule moléculaire : C₃₉H₅₇N₁₁O₁₂S ;
HPLC : t_{R} = 21,50 min (gradient : 5-50% MeCN/H₂O + 0,1%TFA en 30 min détection DAD) ;
Détection UV (λ= 276 nm) ;
MS (MALDI-TOF) : *m*/*z* obtenue = 904.5 ([MH]⁺ calculée pour C₃₉H₅₇N₁₁O₁₂S = 904.4).

### Exemple 2 :

### Utilisations du peptide C^{α}amide de formule générale (II)

### 1) Dans une réaction de thioestérification en milieu acide.

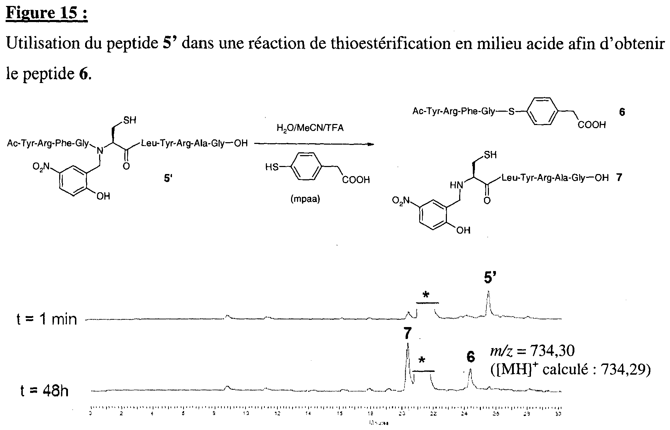

L'utilisation d'un peptide C^{α}-amide **5'** de formule générale (II) dans une réaction de thioestérification est illustrée à la figure 15.

2.5 µmol de peptide **5'** (peptide de formule générale (II)) et 100 µmol d'acide mercaptophénylacétique (à savoir un thiol de formule générale R'-SH avec R' représentant pHOOC-CH₂-C₆H₄- tel que représenté à la figure 15) sont dissouts dans 1 mL du mélange H₂O/MeCN (7:3) + 0,1% TFA. Le mélange réactionnel est agité pendant 48 heures à température ambiante. L'avancement de la réaction est suivi par HPLC (figure 15).

On obtient ainsi le peptide C^{α}-thioester recherché de formule **6** et le composé **7** répondant à la formule générale (I bis) dans laquelle :

X représente S, R₁ représente H, R₃, R₄ et R₅ représentent H, R₂₀ représente H, m est égal à n qui est égal à 0, R₂ représente -CO-Leu-Tyr-Arg-Ala-Gly-OH, et A représente :

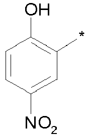

Ce composé de formule (I bis) est particulièrement avantageux du fait de son absorbtion caractéristique dans l'ultraviolet (λmax ∼ 400-450 nm) : une analyse par HPLC avec détection dans cette gamme de longueur d'onde permet de le détecter et de le caractériser facilement ce qui permettra également à l'homme de l'art de conclure que le peptide C^{α}-thioester de formule (IX) tel que défini ci-dessus a bien été formé.

Peptide C^{α}-thioester **6** (figure 15) :

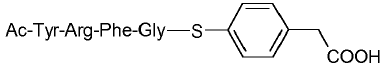

### 2) Dans une réaction de ligation chimique native NCL

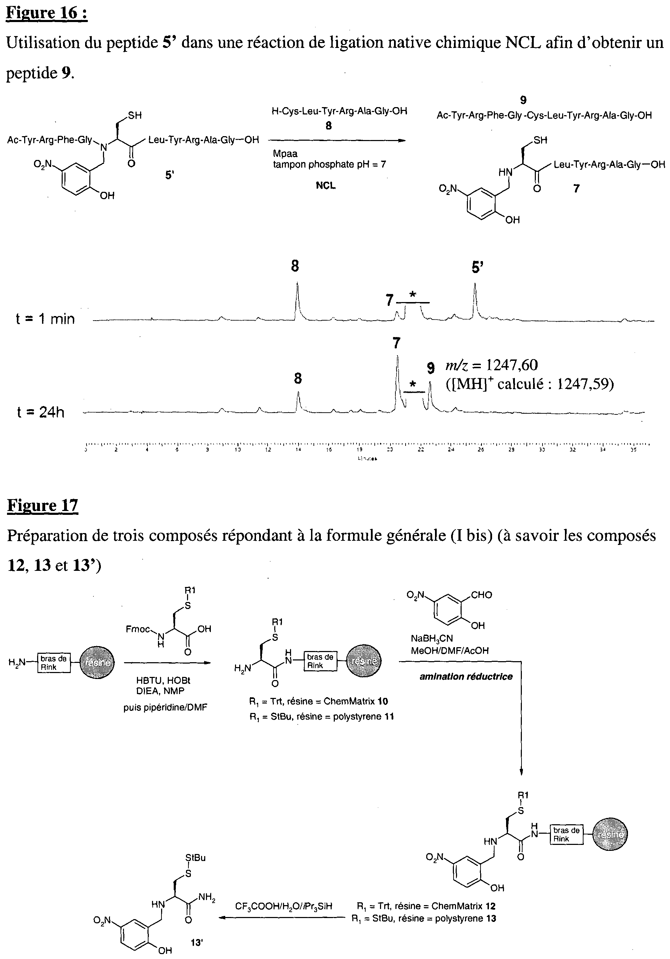

L'utilisation d'un peptide C^{α}-amide **5'** de formule générale (II) dans une réaction de ligation chimique native est illustrée à la figure 16.

Le peptide C^{α}-amide de formule générale (II) (composé **5'**), de par ses propriétés de crypto-thioester, peut être utilisé directement dans une réaction de ligation chimique native NCL, sans le convertir préalablement en peptide C^{α}-thioester (figure 16).

2.5 µmol de peptide cryptothioester brut **5'** et 1.5 équivalents de peptide cystéinyle **8** sont dissouts dans 1 mL de solution dégazée contenant 50 mM d'acide mercaptophénylacétique, 20 mM de triscarboxyéthylphosphine et 200 mM de tampon phosphate pH=7. Le mélange réactionnel est agité pendant 24h à 37°C sous argon.

On obtient ainsi un peptide **9** et un composé **7** de formule générale (I bis). Peptide **9** (figure 16) : Ac-Tyr-Arg-Phe-Gly-Cys-Leu-Tyr-Arg-Ala-Gly-OH

Dans les exemples 3 et 4 décrits ci-dessous, dans le radical de formule (I) ou dans le composé (I bis):
X représente un atome de soufre,
R₁ représente un atome d'hydrogène, un groupe trityle (Trt), ou un groupe *tert-*butylsulfanyle (StBu),
R₂ représente un groupe -B-C-D dans lequel :
   B représente -CO-NH-, avec
   C est absent et D représente un atome d'hydrogène, ou,
   C représente un bras de Rink (-CH[2,4-di-MeO-C₆H₄]-C₆H₄-O-CH₂-CO-NH-CH₂) et D représente un support solide tel qu'une résine de type ChemMatrix ou polystyrène,
R₃, R₄ et R₅ représentent chacun un atome d'hydrogène,
m est égal à n qui est égal à 0,
A représentant un groupe aryle de formule générale : 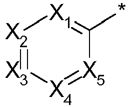 dans laquelle X₁ représente C-OH, chacun des X₂, X₃ et X₅ représente CH et X₄ représente C-NO₂,
et, si il s'agit du composé (I bis), R₂₀ représente un atome d'hydrogène.

Lorsque D représente un atome d'hydrogène cela signifie que le radical (I) n'est pas accroché sur un support solide. Dans ce cas R₁ représente H ou un groupe *tert-*butylsulfanyle (StBu) et R₂ représente un groupe -B-C-D dans lequel B représente - CO-NH-, C est absent et D représente un atome d'hydrogène.

Lorsque D représente un support solide cela signifie que le radical (I) est accroché sur ledit support solide (ledit support solide faisant partie intégrante de la définition du radical (I)). Dans ce cas R₁ représente un groupe trityle (Trt) ou un groupe *tert*-butylsulfanyle (StBu) et R₂ représente un groupe -B-C-D dans lequel B représente -CO-NH-, C représente un bras de Rink (-CH[2,4-di-MeO-C₆H₄]-C₆H₄-O-CH₂-CO-NH-) et D représente un support solide tel qu'une résine de type ChemMatrix ou polystyrène.

Les produits obtenus ont été analysés par HPLC sur Colonne : nucleosil C18 300A 5µm, 4,6x250 mm, gradient : 5-50% MeCN/H₂O + 0,1% TFA en 30 min, débit : 1 ml/min, suivie d'une détection UV (λ=276 nm ou 320 nm) puis d'une spectrométrie de masse MALDI-TOF.

### Exemple 3 :

### Préparation de peptides C^{α}amide de formule générale (II)

### 1) Synthèse d'un composé de formule générale (I bis)

La synthèse d'un composé de formule générale (I bis) est illustrée à la figure 17.

Les composés **12**, **13** et **13'** répondent à la formule générale (I bis) dans laquelle :
→ pour les composés **12** et **13**, X représente S, R₃, R₄ et R₅ représentent H, m est égal à n qui est égal à 0, R₂₀ représente un atome d'hydrogène,
   R₂ représente -CO-NH-bras de Rink-Résine, A représente : 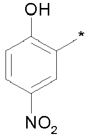 et R₁ représente Trt (**12**), ou StBu (**13**)
→ pour le composé **13'**, X représente S, R₁ représente StBu, R₃, R₄ et R₅ représentent H, m est égal à n qui est égal à 0, R₂₀ représente un atome d'hydrogène, R₂ représente -CO-NH₂, A représente :

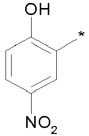

### • Synthèse des cystéinyle résines 10 et 11

Le schéma de synthèse est illustré à la figure 17.

Les composés **10** et **11** répondent à la formule générale (IV bis) telle que définie ci-dessus : 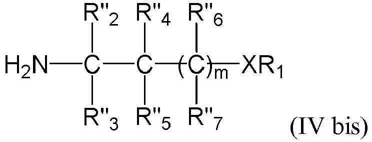 dans laquelle
X représente S, R"₃, R"₄, R"₅, R"₆ et R"₇ représentent H, m est égal à 0, R"₂ représente -NH-bras de Rink-Résine et R₁ représente Trt (**10**) ou StBu (**11**).

Les cystéinyle résines **10** et **11** de formule respective :
H-Cys(Trt)-NH-bras de Rink-Résine (**10**) et
H-Cys(StBu)-NH-bras de Rink-Résine (**11**),
ont été synthétisées dans des conditions standard (synthétiseur ABI 433, programme Fastmoc).

### • Aminations réductrices

Le schéma de synthèse est illustré à la figure 17.

0,1 mmol de la peptidyle résine **10** ou **11** telle que définie respectivement ci-dessus est solvatée dans le mélange diméthylformamide/méthanol/acide acétique (9 :9 :2).

167 mg (10 équivalents) de 2-hydroxy-5-nitrobenzaldehyde dissouts dans 4 mL de diméthylformamide /méthanol (1 :1) sont ajoutés à la résine. La suspension ainsi obtenue est agitée pendant 45 minutes. La résine est ensuite lavée avec le mélange diméthylformamide /méthanol (1 :1).

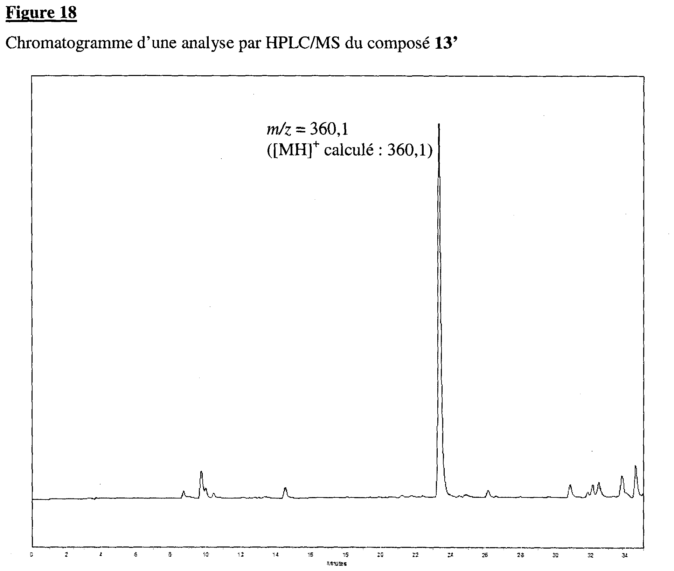

126 mg (20 équivalents) de NaBH₃CN préalablement dissouts dans 4 mL de diméthylformamide /méthanol/acide acétique (9 :9 :2) sont ajoutés à la résine. La suspension ainsi obtenue est agitée pendant 30 minutes. La résine est lavée avec le mélange diméthylformamide /méthanol/acide acétique (9 :9 :2). Une aliquote de la résine **13** est traitée pendant 2h par TFA/H₂O/*i*-Pr₃SiH 92,5:5:2,5 afin d'obtenir le composés **13'**, qui est analysé par HPLC et spectrométrie de masse après évaporation du TFA (voir figure 18).

Composé **13'** (figure 18):
Formule brute : C₁₄H₂₁N₃O₄S₂,
HPLC : t_{R} = 23,4 min (C18, gradient : 5-50% MeCN/H₂O + 0,1%TFA en 30 min) ;
Détection UV (λ= 320 nm) ;
MS (ESI+) : *m*/*z* observée = 360,1 ([MH]⁺ calculée pour C₁₄H₂₂N₃O₄S₂ = 360,1).

### 2) Synthèse d'un peptide C^{α}amide de formule générale (II)

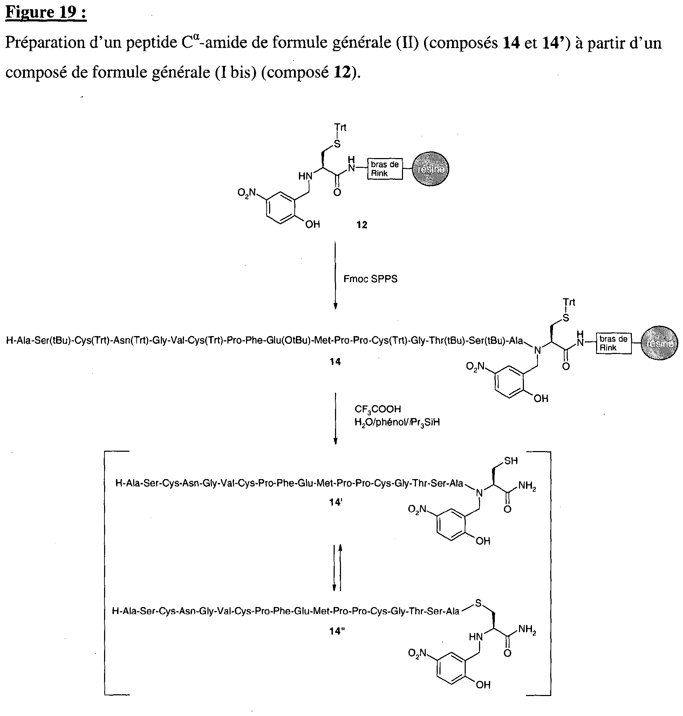

La synthèse d'un peptide C^{α}-amide de formule générale (II) est illustrée à la figure 19. Les composés **14**, **14'**, **15** et **15'** répondent à la formule générale (II) dans laquelle :
→ pour le composé **14**, X représente S, R₁ représente Trt, R₃, R₄ et R₅ représentent H, Peptide 1 représente H-Ala-Ser(tBu)-Cys(Trt)-Asn(Trt)-Gly-Val-Cys(Trt)-Pro-Phe-Glu(OtBu)-Met-Pro-Pro-Cys(Trt)-Gly-Thr(tBu)-Ser(tBu)-Ala-, m est égal à n qui est égal à 0, R₂ représente -CO-NH-bras de Rink-Résine, et A représente : 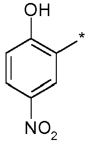
→ pour le composé **14'**, X représente S, R₁ représente H, R₃, R₄ et R₅ représentent H, Peptide 1 représente H-Ala-Ser-Cys-Asn-Gly-Val-Cys-Pro-Phe-Glu-Met-Pro-Pro-Cys-Gly-Thr-Ser-Ala-, m est égal à n qui est égal à 0,
   R₂ représente -CO-NH₂, et A représente : 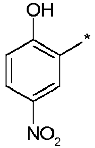
→ pour le composé **15**, X représente S, R₁ représente StBu, R₃, R₄ et R₅ représentent H, Peptide 1 représente H-Leu-Tyr(tBu)-Arg(Pbf)-Ala-Gly-, m est égal à n qui est égal à 0, R₂ représente -CO-NH-bras de Rink-Résine, et A représente 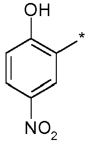
→ pour le composé **15'**, X représente S, R₁ représente StBu, R₃, R₄ et R₅ représentent H, Peptide 1 représente H-Leu-Tyr(tBu)-Arg(Pbf)-Ala-Gly-, m est égal à n qui est égal à 0, R₂ représente -CO-NH₂, et A représente :

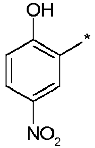

### • Elongation du peptide 14' à partir du composé 12

L'élongation du peptide **14'** est représentée à la figure 19.

L'élongation du peptide est réalisée dans des conditions standard (ABI 433, programme Fastmoc, HCTU comme agent de couplage) avec la résine **12**, en effectuant deux fois de suite le couplage du premier acide aminé Fmoc-Ala-OH avant de déprotéger le groupement Fmoc et de continuer l'élongation.

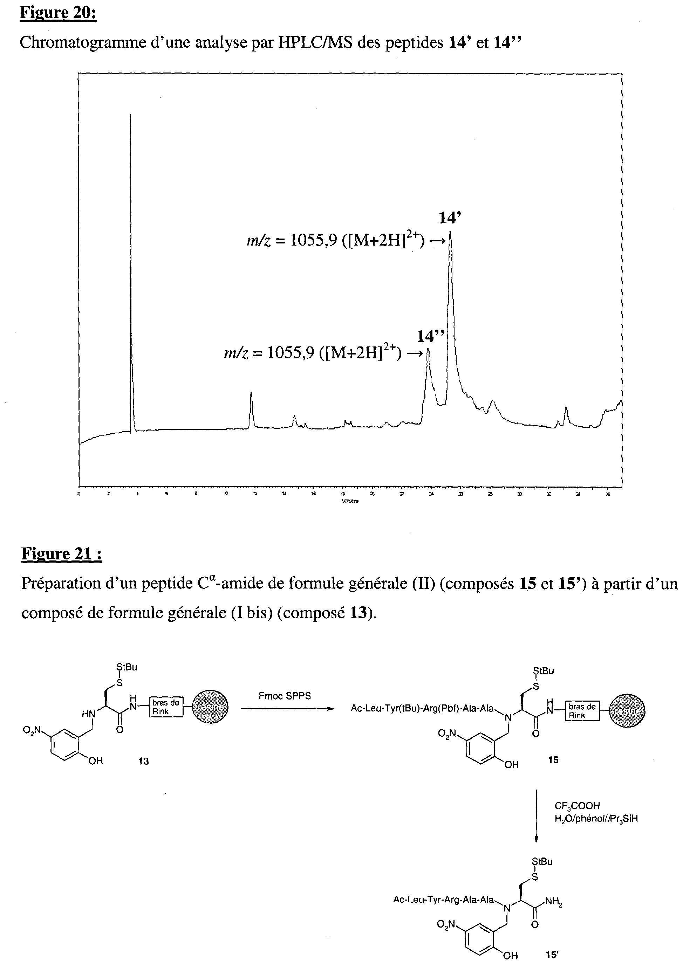

La peptidyle résine **14** obtenue est traitée pendant 2h par TFA/PhOH/H₂O/*i-*Pr₃SiH 87,5:5:5:2,5 pour obtenir le peptide **14'** qui est précipité par dilution dans un mélange éther de pétrole/Et₂O 1 :1, lavé (Et₂O) puis séché sous pression réduite. Le précipité est repris dans de l'eau déminéralisée et lyophilisé. Il est ensuite analysé par HPLC et spectrométrie de masse (figure 21). Le peptide amide **14'** est obtenu en mélange avec le peptide thioester **14"** (les deux composés sont en équilibre en solution).

Peptide **14'** (figure 20) :
Formule moléculaire : C₈₅H₁₂₆N₂₃O₃₁S₅ ;
HPLC : (gradient : 5-50% MeCN/H₂O + 0,1% TFA en 30 min détection UV, λ = 276 nm) - t_{R} = 25,3 min ;
MS (ESI+) : *m*/*z* obtenue = 1055,9 ([M+2H]²⁺).

Peptide **14"** (figure 20) :
Formule moléculaire : C₈₅H₁₂₆N₂₃O₃₁S₅ ;
HPLC : t_{R} = 23,8 min (gradient : 5-50% MeCN/H₂O + 0,1% TFA en 30 min ;
Détection UV (λ, = 276 nm) ;
MS (ESI+) : *m*/*z* obtenue = 1055,9 ([M+2H]²⁺).

### • Elongation du peptide 15' à partir du composé 13

L'élongation du peptide **15'** est représentée à la figure 21.

L'élongation du peptide est réalisée dans des conditions standard (ABI 433, programme Fastmoc, HCTU comme agent de couplage) avec la résine **13**, en effectuant deux fois de suite le couplage du premier acide aminé Fmoc-Ala-OH avant de déprotéger le groupement Fmoc et de continuer l'élongation.

La peptidyle résine **15** obtenue est traitée pendant 2h par TFA/*i*-Pr₃SiH 97,5:5:5:2,5 pour obtenir le peptide **15'** qui est précipité par dilution dans un mélange éther de pétrole/Et₂O 1 : 1, lavé (Et₂O), séché puis analysé par HPLC et spectrométrie de masse (figure 21).

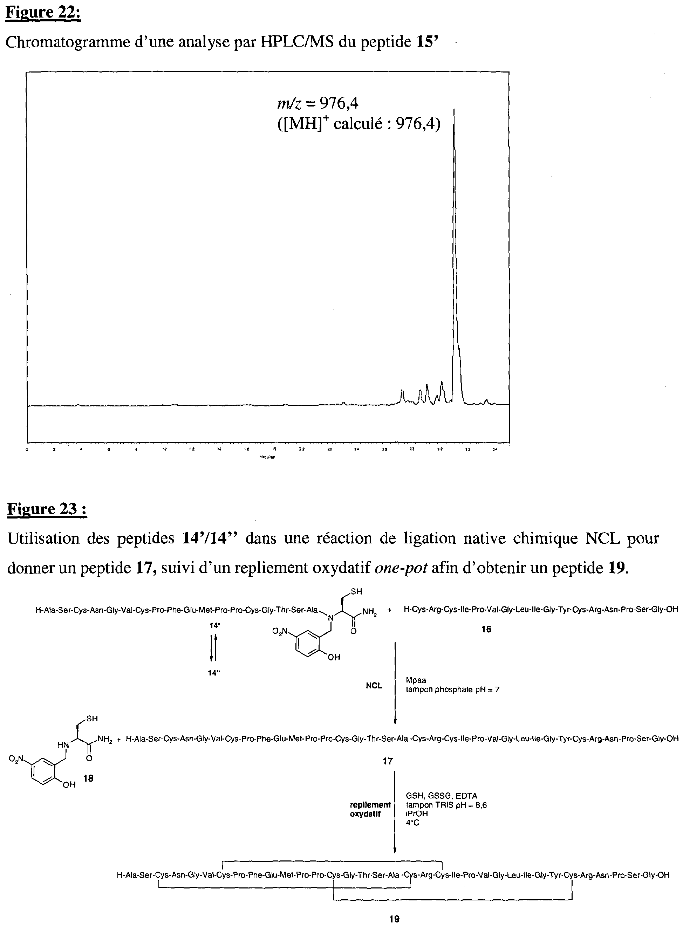

Peptide **15'** (figure 22) :
Formule moléculaire : C₄₃H₆₅N₁₁O₁₁S₂ ;
HPLC : t_{R} = 31.0 min (gradient : 5-50% MeCN/H₂O + 0,1% TFA en 30 min) ;
Détection UV, λ, = 320 nm) ;
MS (MALDI-TOF) : *m*/*z* obtenue = 976,4 ([MH]⁺ calculée pour C₄₃H₆₅N₁₁O₁₁S₂ = 976,4).

### Exemple 4 :

### Utilisations du peptide C^{α}-amide de formule générale (II) dans une réaction de ligation chimique native (NCL)

L'utilisation d'un peptide C^{α}-amide **14'** de formule générale (II) dans une réaction de ligation chimique native est illustrée à la figure 23.

Le peptide C^{α}-amide de formule générale (II) (composé **14'**), de par ses propriétés de crypto-thioester, peut être utilisé directement dans une réaction de ligation chimique native NCL, sans le convertir préalablement en peptide C^{α}-thioester (figure 23).

### • Préparation du partenaire cystéinyle peptide 16

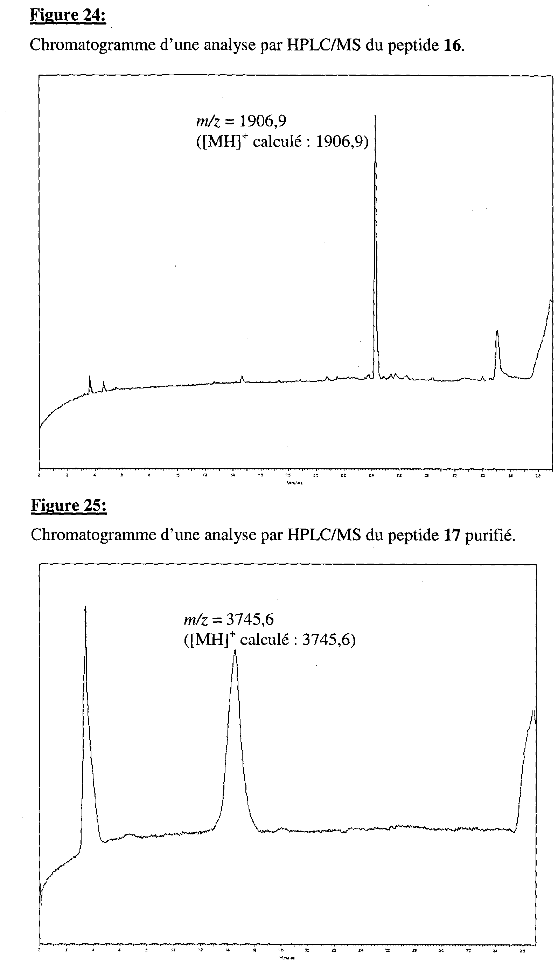

Le peptide **16** (H-CRCIPVGLIGYCRNPSG-OH) a été synthétisé dans des conditions standard (synthétiseur ABI 433, programme Fastmoc, agent de couplage : HCTU). La résine obtenue après élongation automatisée est traitée pendant 2h par TFA/PhOH/H₂O/*i*-Pr₃SiH 87,5:5:5:2,5 pour décrocher et déprotéger le peptide **16**, qui est précipité par dilution dans un mélange éther de pétrole/Et₂O 1:1, lavé (Et₂O) puis séché. Le précipité est repris dans de l'eau déminéralisée puis lyophilisé. Il est ensuite analysé par HPLC et spectrométrie de masse (figure 24).

Peptide **16** (figure 24) :
Formule moléculaire : C₈₁H₁₃₅N₂₅O₂₂S₃ ;
HPLC : t_{R} = 24,3 min (gradient : 5-50% MeCN/H₂O + 0,1% TFA en 30 min) Détection UV (λ = 276 nm) ;
MS (MALDI-TOF) : *m*/*z* obtenue = 1906,9 ([MH]+ calculée pour C₈₁H₁₃₆N₂₅O₂₂S₃= 1906,9).

### • Ligation chimique native entre les peptides 14'/14" et 16

2.5 µmol de peptide cryptothioester brut **14'/14"** et 1.5 équivalents de cystéinyle peptide brut **16** sont dissouts dans 1 mL d'une solution déoxygénée contenant 25 mM d'acide mercaptophénylacétique, 50 mM de triscarboxyéthylphosphine et 200 mM de tampon phosphate pH=7. Le mélange réactionnel est agité pendant 3h à 37°C sous une atmosphère d'argon. On obtient ainsi un peptide **17** et un composé **18** de formule générale (I bis). Le peptide **17** est purifié par HPLC semi-préparative puis lyophilisé. Il est obtenu avec un rendement de 65%.

Peptide **17** (figure 25) :
Formule moléculaire : C₁₅₆H₂₄₉N₄₅O₄₈S₇ ;
HPLC : t_{R} = 14,6 min (gradient : 30-45% MeCN/H₂O + 0,1% TFA en 30 min) ;
Détection UV (λ = 276 nm) ;
MS (MALDI-TOF) : *m*/*z* obtenue = 3745,6 ([M+H]⁺ calculée pour C₁₅₆H₂₅₀N₄₅O₄₈S₇ = 3745,6).

### • Ligation chimique native entre les peptides 14'/14" et 16 suivi d'un repliement oxydatif one-pot

2,5 µmol de peptide cryptothioester brut **14'/14"** et 3,75 µmol (1.5 équivalents) de cystéinyle peptide brut **16** sont dissouts dans 1 mL d'une solution déoxygénée contenant 25 mM d'acide mercaptophénylacétique, 50 mM de triscarboxyéthylphosphine et 200 mM de tampon phosphate pH=7. Le mélange réactionnel est agité pendant 3h à 37°C sous une atmosphère d'argon. Le mélange réactionnel est directement engagé dans une étape de repliement oxydatif du produit de la ligation **17** (concentration finale en **17** : 13µM) par dilution dans un mélange 1 :1 d'isopropanol et d'un tampon contenant 0.2 mM de Tris pH 8.6, 2 mM d'EDTA, 1,82 mM de glutathion (GSH) et 0,78 mM de glutathion oxydé (GSSG), et agitation de la solution résultante pendant 24 heures à 4°C. La mini-protéine à trois ponts disulfure **19** est purifiée par HPLC semi-préparative puis lyophilisée. **19** est obtenu avec un rendement de 55%.

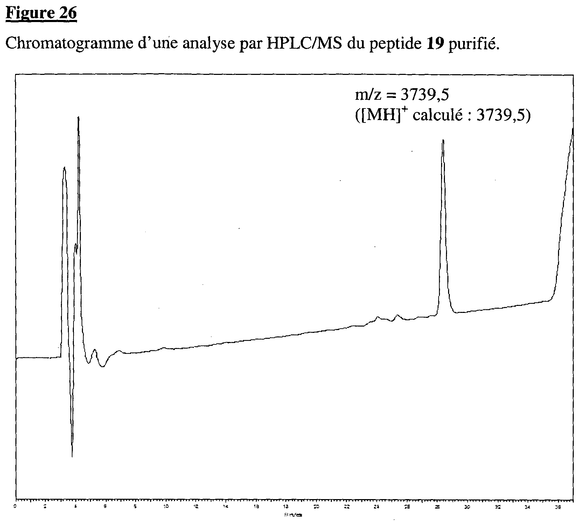

Peptide **19** (figure 26) :
Formule moléculaire : C₁₅₆H₂₄₃N₄₅O₄₈S₇ ;
HPLC : t_{R} = 28,4 min (gradient : 30-45% MeCN/H₂O + 0,1% TFA en 30 min) ;
Détection UV (λ = 276 nm) ;
MS (MALDI-TOF) : *m*/*z* obtenue = 3739,5 ([MH]+ calculée pour C₁₅₆H₂₄₄N₄₅O₄₈S₇ = 3739,5).

## Revendications

1. Peptide C^{α}-amide précurseur d'un peptide C^{α}-thioester **caractérisé en ce qu'**il comprend le radical de formule générale (I) : 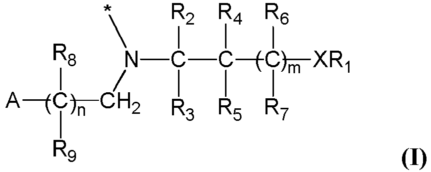 dans laquelle
X représente un atome de soufre ou de sélénium,
R₁ représente un atome d'hydrogène ou un groupe protecteur du soufre ou du sélénium compatible avec des conditions d'élongation par Fmoc SPPS,
m représente un entier égal à 0 ou 1,
un des R₂, R₃, R₄, R₅, R₆ ou R₇ représente le radical -B-C-D dans lequel :
D représente un atome d'hydrogène ou un support solide convenant pour la synthèse de peptides en phase solide (SPPS),
C est absent ou représente un bras utilisable pour la SPPS,
B représente un radical divalent comportant un hétéroatome,
les autres desdits R₂, R₃, R₄, R₅, R₆ ou R₇ qui ne représentent pas -B-C-D représentent alors indépendamment l'un de l'autre un atome d'hydrogène, un radical alkyle ayant de 1 à 5 atomes de carbone, et de préférence un radical méthyle (-CH₃) ou un radical phényle (-C₆H₅), à la condition qu'au plus deux desdits radicaux R₂, R₃, R₄, R₅, R₆ ou R₇ représentent en même temps un phényle,
R₈ et R₉ représentent indépendamment l'un de l'autre un radical alkyle ayant de 1 à 10 atomes de carbone, et de préférence un radical méthyle ou un atome d'hydrogène,
n représente un entier allant de 0 à 4, de préférence de 0 à 1,
A représentant un radical aryle ou hétéroaryle choisi dans le groupe comprenant
• le radical de formule : 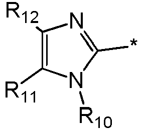
dans laquelle
R₁₀ représente un radical alkyle ayant de 1 à 10 atomes de carbone, et de préférence un méthyle,
R₁₁ et R₁₂ représentent indépendamment l'un de l'autre un atome d'hydrogène, un atome d'halogène choisi dans le groupe comprenant Cl, Br, I ou F, un radical -CN, un radical -NO₂, un radical -CF₃, un radical phényle (-C₆H₅), un radical -CONH₂, un radical R₁₀, un radical -OR₁₀, un radical -SR₁₀, un radical -N(R₁₀)₂, un radical -COOR₁₀, un radical -CONHR₁₀ ou un radical -CON(R₁₀)₂, R₁₀ étant tel que défini précédemment,
• le radical de formule : 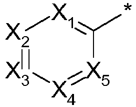 dans laquelle
au moins un des X₁, X₂, X₃, X₄ et X₅ représente un atome d'azote (N), un radical C-OH ou un radical C-SH, les autres desdits X₁, X₂, X₃, X₄ ou X₅ qui ne représentent pas N, C-OH ou C-SH représentent alors indépendamment l'un de l'autre un radical C-R₁₁ avec R₁₁ tel que défini précédemment,
• le radical de formule : 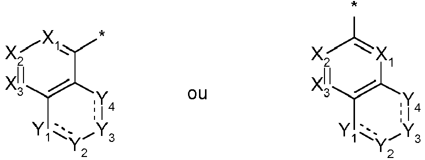
dans laquelle
au moins un des X₁, X₂, X₃ et Y₄ représente un atome d'azote (N), un radical C-OH ou un radical C-SH, les autres desdits X₁, X₂ ou X₃ qui ne représentent pas N, C-OH ou C-SH représentent alors indépendamment l'un de l'autre un radical C-R₁₁ avec R₁₁ tel que défini précédemment,
Y₁, Y₂, Y₃ et Y₄ (à condition que Y₄ ne représente pas C-OH ou C-SH) représentent indépendamment l'un de l'autre, selon qu'ils soient liés par une liaison simple ou double, un atome d'azote (N) ou un groupe NR₁₀ avec R₁₀ tel que défini précédemment, un groupe CH ou CH₂, un groupe C-R₁₁ ou CHR₁₁ ou CR₁₁R₁₂ avec R₁₁ et R₁₂ tels que définis précédemment, un carbonyle (C=O), un atome d'oxygène (O), un atome de soufre (S), avec la condition qu'au plus deux desdits Y₁, Y₂, Y₃ et Y₄ représentent en même temps un atome d'oxygène ou un atome de soufre,
l'un desdits Y₁, Y₂, Y₃ ou Y₄ pouvant être absent, de sorte à former un cycle à 5 chaînons.

2. Peptide C^{α}-amide selon la revendication 1, **caractérisé en ce que** dans le radical (I), le radical divalent B représente : 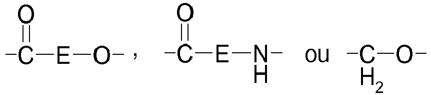 dans lequel
E est absent ou représente le groupe -(Xaa)ᵢ- dans lequel :
i représente un entier allant de 1 à 20, et de préférence de 1 à 5,
chaque Xaa représente indépendamment les uns des autres un résidu d'acide aminé, et lorsque i est supérieur ou égal à 2, chacun desdits Xaa est relié à son voisin Xaa par une liaison peptidique,
ou, dans le cas où m =1, et R₄ ou R₅ = -B-C-D, alors le radical divalent B représente de préférence : 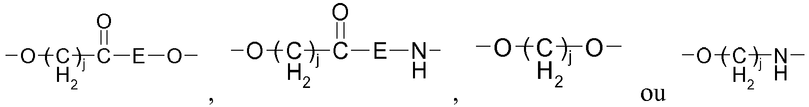 dans lequel :
j représente un entier allant de 0 à 10, de préférence de 0 à 3 et E est tel que défini précédemment.

3. Peptide C^{α}-amide selon la revendication 1 ou la revendication 2, **caractérisé en ce que** dans le radical (I), C représente un bras utilisable pour la SPPS en stratégie Fmoc.

4. Peptide C^{α}-amide selon la revendication 3, **caractérisé en ce que** C représente un bras acido-labile choisi dans le groupe comprenant un bras de Rink (4-[(2,4-diméthoxyphényl)méthyl]phénoxyacétyle), un bras de type Wang (4-alkoxybenzyle), un bras de Sieber (xanthén-3-yloxyalkyle), un bras PAL (4-2,5-diméthoxy-alkoxybenzyle), un bras 2-chlorotrityle, un bras PAM (phénylacetamidométhyle), un bras SASRIN (2-méthoxy-4-alkoxybenzyle) ou un bras MBHA (4-méthyl)benzhydryle.

5. Peptide C^{α}-amide selon l'une quelconque des revendications 1 à 4, **caractérisé en ce que** dans le radical (I), D représente un support solide convenant pour la SPPS en stratégie Fmoc et de préférence est choisi dans le groupe comprenant une résine commercialisée sous la dénomination PEGA®, une résine commercialisée sous la dénomination CHEMMATRIX™, une résine de type polyacrylamide, une résine de type polystyrène ou une résine mixte polystyrène/polyéthylèneglycol (PEG).

6. Peptide C^{α}-amide selon l'une quelconque des revendications 1 à 5, **caractérisé en ce que** dans le radical (I), n est un entier égal à 0, et le radical A présente la formule : 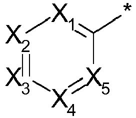 dans laquelle X₁ représente C-OH, et chacun des X₂, X₃, X₄ et X₅ est tel que défini à la revendication 1.

7. Peptide C^{α}-amide selon la revendication 6, **caractérisé en ce que** dans le radical A, chacun des X₂, X₃ ou X₅ représente CH et X₄ représente C-R₁₁ avec R₁₁ tel que défini à la revendication 1, et de préférence X₄ représente CH, C-OMe ou C-NO₂.

8. Peptide C^{α}-amide selon l'une quelconque des revendications 1 à 5, **caractérisé en ce que** dans le radical (I), n est un entier égal à 0, et le radical A présente la formule : 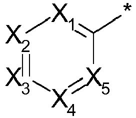 dans laquelle X₁ représente l'atome d'azote (N) et chacun desdits X₂, X₃, X₄ et X₅ est tel que défini à la revendication 1.

9. Peptide C^{α}-amide selon la revendication 8, **caractérisé en ce que** dans le radical A, chacun des X₂, X₃ ou X₅ représente CH et X₄ représente CH ou C-R₁₁ avec R₁₁ représentant OCH₃ ou N(CH₃)₂.

10. Peptide C^{α}-amide selon l'une quelconque des revendications 1 à 5, **caractérisé en ce que** dans le radical (I), n est un entier égal à 0, et le radical A présente la formule : 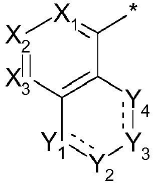 dans laquelle X₁ ou Y₄ représente C-OH ou N, l'autre desdits X₁ ou Y₄ qui ne représente pas C-OH ou N, ainsi que chacun desdits X₂, X₃, Y₁, Y₂, et Y₃ sont tels que définis à la revendication 1.

11. Peptide C^{α}-amide selon l'une quelconque des revendications 1 à 5, **caractérisé en ce que** dans le radical (I), n est un entier égal à 0, et le radical A présente la formule : 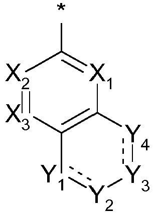 dans laquelle X₁ ou X₂ représente C-OH ou N, l'autre desdits X₁ ou X₂ qui ne représente pas C-OH ou N, ainsi que chacun desdits X₃, Y₁, Y₂, Y₃ et Y₄ sont tels que définis à la revendication 1.

12. Peptide C^{α}-amide selon l'une quelconque des revendications 1 à 11, **caractérisé en ce que** dans le radical (I) :
m représente 0 ou 1,
chacun desdits R₃, R₄, R₅, R₆ ou R₇ représente H,
R₂ représente -B-C-D dans lequel B représente : 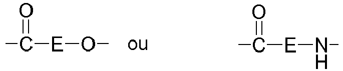
et C, D et E sont tels que définis à l'une quelconque des revendications 1 à 4.

13. Peptide C^{α}-amide selon l'une quelconque des revendications 1 à 12, **caractérisé en ce que** dans le radical (I), m est égal à 0 et X représente un atome de soufre (S).

14. Peptide C^{α}-amide selon l'une quelconque des revendications 1 à 13, **caractérisé en ce que** dans le radical (I), R₁ est un groupe protecteur du soufre choisi dans le groupe comprenant trityle (Trt), méthyltrityle (Mtt), méthoxytrityle (Mmt), xanthényl (Xan), tri-méthoxybenzyle (Tmob), acétamidométhyle (Acm), triméthylacétamidométhyle (Tacm), benzamidométhyle (Bam), allyloxycarbonylaminométhyle (Allocam), phtalimidométhyle (Pim), 2-(triméthylsilyl)éthyle, t-butyle (tBu), 2,4,6-triméthylbenzyle, quinolinylméthyle (Qm), diphényl-4-pyridylméthyle, 1-adamantyle, benzyloxyméthyle (BOM), 2-tétrahydropyranyle (Thp), benzylthiométhyle, éthylsulfényle (SEt), t-butylsulfényle (StBu), phénylsulfényle (SPh) et 2,4-dinitrophényle.

15. Peptide C^{α}-amide selon l'une quelconque des revendications 1 à 14, **caractérisé en ce que** ledit peptide présente la formule générale (II) : 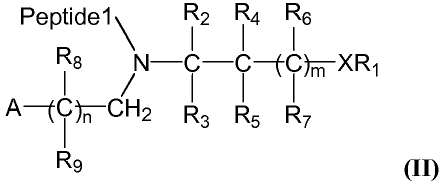 dans laquelle :
Peptide1 représente le groupement R₁₈-(Xaa)ₖ- dans lequel :
k est un entier allant de 1 à 100, de préférence de 4 à 60, et plus préférentiellement encore de 8 à 40,
Xaa représente indépendamment les uns des autres un résidu d'acide aminé provenant d'un acide aminé de formule H-Xaa-OH, et lorsque k est supérieur ou égal à 2, chacun desdits Xaa est relié à son voisin Xaa par une liaison peptidique,
R₁₈ est un atome d'hydrogène ou un substituant de l'extrémité N-terminale comprise dans le résidu Xaa,
X, m, n, A et R₁ à R₉ étant tels que définis à la revendication 1.

16. Composé **caractérisé en ce qu'**il présente la formule générale (I bis) : 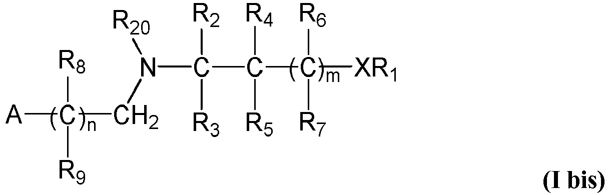 dans laquelle :
X, R₁, m, R₂, R₃, R₄, R₅, R₆, R₇, n, R₈, R₉ et A sont tels que définis à la revendication 1 et R₂₀ représente R₁₄,
avec R₁₄ = R₁₃ et R₁₃ représente un groupement protecteur de la fonction amine, et de préférence un groupement Fmoc,
ou R₁₄ représente un résidu amino acyle protégé de formule R₁₃-Xaa- avec Xaa représentant un résidu d'acide aminé provenant d'un acide aminé de formule H-Xaa-OH.

17. Composé **caractérisé en ce qu'**il présente la formule générale (I bis) : 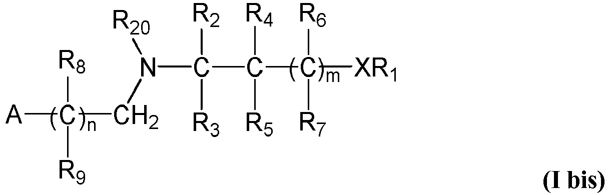 dans laquelle :
X, R₁, m, n, R₈, R₉ et A sont tels que définis à la revendication 1,
un des R₂, R₃, R₄, R₅, R₆ ou R₇ représente le radical -B-C-D dans lequel :
D représente un atome d'hydrogène ou un support solide convenant pour la synthèse de peptides en phase solide (SPPS),
C est absent ou représente un bras utilisable pour la SPPS,
B représente : 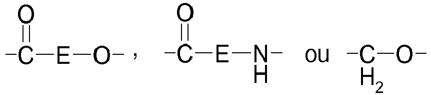
dans lequel
E est absent ou représente le groupe -(Xaa)ᵢ- dans lequel :
i représente un entier allant de 1 à 20, et de préférence de 1 à 5,
chaque Xaa représente indépendamment les uns des autres un résidu d'acide aminé, et lorsque i est supérieur ou égal à 2, chacun desdits Xaa est relié à son voisin Xaa par une liaison peptidique,
ou, dans le cas où m =1, et R₄ ou R₅ = -B-C-D, alors le radical divalent B représente de préférence : 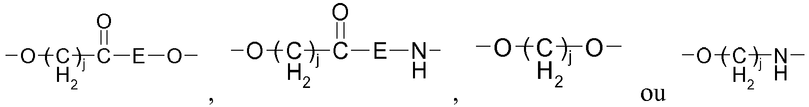
dans lequel :
j représente un entier allant de 0 à 10, de préférence de 0 à 3 et E est tel que défini précédemment,
les autres desdits R₂, R₃, R₄, R₅, R₆ ou R₇ qui ne représentent pas -B-C-D représentent alors indépendamment l'un de l'autre un atome d'hydrogène, un radical alkyle ayant de 1 à 5 atomes de carbone, et de préférence un radical méthyle (-CH₃) ou un radical phényle (-C₆H₅), à la condition qu'au plus deux desdits radicaux R₂, R₃, R₄, R₅, R₆ ou R₇ représentent en même temps un phényle,
R₂₀ représente un hydrogène ou R₁₄,
avec R₁₄ = R₁₃ et R₁₃ représente un groupement protecteur de la fonction amine, et de préférence un groupement Fmoc,
ou R₁₄ représente un résidu amino acyle protégé de formule R₁₃-Xaa- avec Xaa représentant un résidu d'acide aminé provenant d'un acide aminé de formule H-Xaa-OH.

18. Procédé de préparation d'un peptide C^{α}-amide de formule (II) tel que défini à la revendication 15 par Fmoc SPPS, **caractérisé en ce qu'**il comporte une étape d'élongation du composé (I bis) tel que défini à la revendication 16 ou 17, ladite étape d'élongation permettant d'ajouter Peptide1 tel que défini à la revendication 15.

19. Procédé de préparation d'un peptide C^{α}-thioester de formule générale (IX) :
Peptide1- S - R' **(IX)**
dans laquelle :
R'- représente un radical provenant d'un thiol de formule R'-SH, et est un groupement alkyle, aryle, arylalkyle, cycloalkyle, hétérocycloalkyle, hétéroaryle, pouvant comporter un ou plusieurs substituants choisis parmi halogène, carboxyle, sulfonate, ammonium, alcool, éther, alkyle, cycloalkyle, hétérocycloalkyle, aryle, hétéroaryle, alcoxy, halogénoalkyle, arylalkyle, hétéroarylalkyle, arylhétérocycloalkyle,
Peptide 1 est tel que défini à la revendication 15,
**caractérisé en ce qu'**il comprend une réaction de thio-estérification entre le peptide C^{α}-amide de formule (II) tel que défini à la revendication 15 dans laquelle R₁ est égal à H, et, un thiol de formule R'-SH,
afin d'obtenir le peptide C^{α}-thioester de formule (IX) tel que défini ci-dessus et, le composé de formule (I ter) : 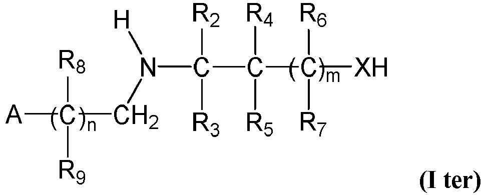
dans laquelle X, m, R₂, R₃, R₄, R₅, R₆, R₇, n, R₈, R₉ et A sont tels que définis à la revendication 1.

20. Procédé de préparation d'un peptide de formule générale (X) :
Peptidel-Yaa-Peptide2-R₁₅ **(X)**
dans laquelle :
Peptide1 est tel que défini à la revendication 15,
Yaa est un résidu d'acide aminé provenant d'un acide aminé de formule H-Yaa-OH choisi dans le groupe comprenant une cystéine, une homocystéine, une β-mercapto-valine, une β-mercapto-leucine, une β-mercapto-isoleucine, une β-mercapto-phénylalanine, une β-mercapto-lysine, une β-mercapto-proline, une γ-mercapto-valine, une γ-mercapto-isoleucine, une γ-mercapto-leucine, une γ-mercapto-lysine, une γ-mercapto-proline, ou un acide aminé substitué sur son atome d'azote N^{α} par un groupement contenant une fonction β- ou γ-amino thiol ledit groupement étant choisi dans le groupe comprenant: 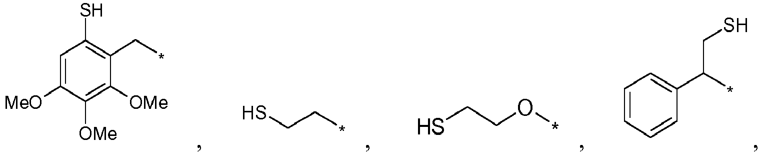 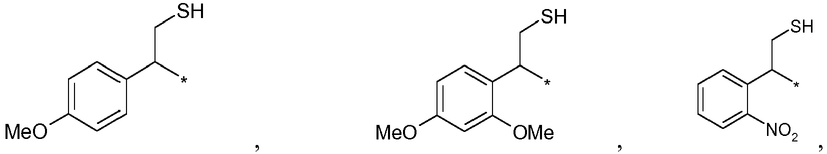 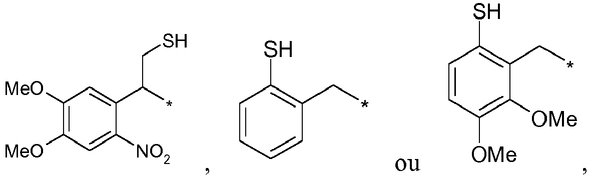
Peptide2= (Xaa)₁, avec 1= un entier allant de 1 à 60, de préférence de 4 à 40, Xaa représente indépendamment les uns des autres un résidu d'acide aminé provenant d'un acide aminé de formule H-Xaa-OH, et lorsque 1 est supérieur ou égal à 2, chacun desdits Xaa étant relié à son voisin Xaa par une liaison peptidique,
R₁₅ représente -OH, -NH₂ ou un radical de formule générale (I) dans laquelle X représente un atompe de soufre et R₁ est un groupe protecteur du soufre qui est stable à un traitement au TFA et stable dans des conditions de NCL,
**caractérisé en ce que** l'on fait réagir par une réaction de NCL :
un peptide C^{α}-amide de formule générale (II) tel que défini à la revendication 15, dans laquelle R1 = H ou un groupement labile dans des conditions de ligation native chimique NCL,
avec un peptide possédant un résidu β- ou γ-amino thiol N terminal de formule générale (XI) :
H-Yaa-Peptide2-R₁₅ **(XI)**
dans laquelle Yaa, R₁₅ et Peptide2 sont tels que définis ci-dessus,
afin d'obtenir
- le peptide de formule générale (X) tel que défini ci-dessus et
- le composé de formule (I bis) tel que défini à la revendication 16 ou à la revendication 17.

21. Utilisation d'un peptide C^{α}-amide tel que défini à l'une quelconque des revendications 1 à 15, pour préparer un peptide C^{α}-thioester.

22. Utilisation d'un peptide C^{α}-amide tel que défini à l'une quelconque des revendications 1 à 15, dans une réaction de ligation native NCL (« Native Chemical Ligation ») pour préparer un peptide ou une protéine, en particulier d'intérêt thérapeutique.

## Patentansprüche

1. C^{α}-Amid-Peptid-Vorläufer eines C^{α}-Thioester-Peptids, **dadurch gekennzeichnet, dass** er den Rest mit der allgemeinen Formel (I) umfasst: 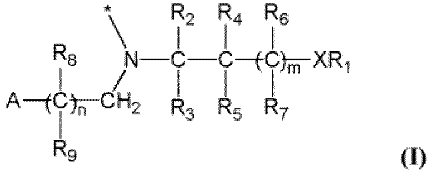 worin,
X für ein Schwefel- oder Selenatom steht,
R₁ für ein Wasserstoffatom oder eine Schutzgruppe des Schwefels oder Selens steht, die kompatibel mit den Bedingungen einer Verlängerung mittels Fmoc-SPPS ist,
m für eine ganze Zahl gleich 0 oder 1 steht,
eines von R₂, R₃, R₄, R₅, R₆ oder R₇ für den Rest -B-C-D steht, wobei:
D für ein Wasserstoffatom oder einen festen Träger steht, der für die Festphasenpeptidsynthese (SPPS) geeignet ist,
C fehlt oder für einen für die SPPS verwendbaren Arm steht,
B für einen zweiwertigen Rest steht, der ein Heteroatom umfasst,
die restlichen R₂, R₃, R₄, R₅, R₆ oder R₇, die nicht für -B-C-D stehen, dann unabhängig voneinander für ein Wasserstoffatom, einen Alkylrest mit 1 bis 5 Kohlenstoffatomen, und vorzugsweise einen Methylrest (-CH₃) oder einen Phenylrest (-C₆H₅) stehen, unter der Vorrausetzung, dass höchstens zwei von R₂, R₃, R₄, R₅, R₆ oder R₇ gleichzeitig für Phenyl stehen, R₈ und R₉ unabhängig voneinander für einen Alkylrest mit 1 bis 10 Kohlenstoffatomen stehen, vorzugsweise für einen Methylrest, oder ein Wasserstoffatom,
n für eine ganze Zahl von 0 bis 4 steht, vorzugsweise von 0 bis 1,
A für einen Aryl- oder Heteroarylrest steht, ausgewählt aus der Gruppe bestehend aus
• dem Rest der Formel: 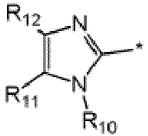 worin,
R₁₀ für einen Alkylrest mit 1 bis 10 Kohlenstoffatomen steht, vorzugsweise Methyl,
R₁₁ und R₁₂ unabhängig voneinander für ein Wasserstoffatom, ein Halogenatom, ausgewählt aus der Gruppe umfassend Cl, Br, I oder F, einen -CN-Rest, einen -NO₂-Rest, einen CF₃-Rest, einen Phenylrest (-C₆H₅), einen -CONH₂-Rest, einen R₁₀-Rest, einen -OR₁₀-Rest, einen -SR₁₀-Rest, einen -N(R₁₀)₂-Rest, einen -COOR₁₀-Rest einen -CONHR₁₀-Rest oder einen -CON(R₁₀)₂-Rest steht, wobei R₁₀ wie oben definiert ist,
• dem Rest der Formel: 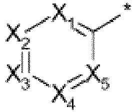 worin
mindestens eines von X₁, X₂, X₃, X₄ und X₅ für ein Stickstoffatom (N), einen C-OH-Rest oder einen C-SH-Rest steht, wobei die restlichen X₁, X₂, X₃, X₄ oder X₅, die nicht für N, C-OH oder C-SH stehen, dann unabhängig voneinander für einen C-R₁₁-Rest stehen, wobei R₁₁ wie oben definiert ist,
• dem Rest der Formel: 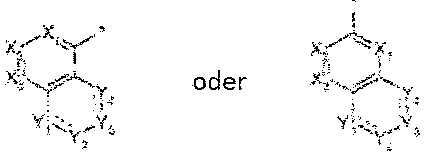 worin
mindestens eines von X₁, X₂, X₃ und Y₄ für ein Stickstoffatom (N), einen C-OH-Rest oder einen C-SH-Rest steht, die restlichen X₁, X₂ oder X₃, die nicht für N, C-OH oder C-SH stehen, dann unabhängig voneinander für einen C-R₁₁-Rest stehen, wobei R₁₁ wie oben definiert ist,
Y₁, Y₂, Y₃ und Y₄ (unter der Voraussetzung, dass Y₄ nicht für C-OH oder C-SH steht), unabhängig voneinander, je nachdem, ob sie durch eine Einfach- oder Doppelbindung gebunden sind, für ein Stickstoffatom (N) oder eine NR₁₀-Gruppe, wobei R₁₀ wie oben definiert ist, eine CH- oder eine CH₂-Gruppe, eine C-R₁₁-Gruppe oder eine CHR₁₁-Gruppe oder eine CR₁₁R₁₂-Gruppe, wobei R₁₁ und R₁₂ wie oben definiert sind, ein Carbonyl (C=O), ein Sauerstoffatom (O), ein Schwefelatom (S), unter der Voraussetzung, dass höchstens zwei von Y₁, Y₂, Y₃ und Y₄ gleichzeitig ein Sauerstoffatom oder ein Schwefelatom darstellen, stehen, wobei eines von Y₁, Y₂, Y₃ oder Y₄ fehlen kann, um einen 5-gliedrigen Ring zu bilden.

2. C^{α}-Amid-Peptid gemäß Anspruch 1, **dadurch gekennzeichnet, dass** im Rest (I), der zweiwertige Rest B für folgendes steht, 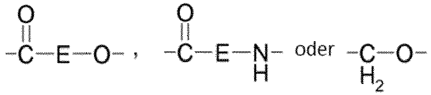 worin
E fehlt oder für eine -(Xaa)ᵢ- -Gruppe steht, worin:
i für eine ganze Zahl von 1 bis 20 steht, vorzugsweise 1 bis 5,
jedes Xaa voneinander unabhängig für einen
Aminosäurerest steht, und wenn i größer als oder gleich 2 ist, jedes Xaa mit dem benachbarten Xaa durch eine Peptidbindung verbunden ist,
oder, in dem Fall, in dem m = 1, und R₄ oder R₅ = -B-C-D, der zweiwertige Rest B dann vorzugsweise für folgendes steht: 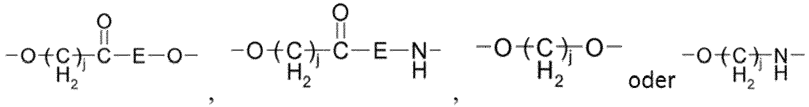 worin:
j für eine ganze Zahl von 0 bis 10 steht, vorzugsweise von 0 bis 3, und E wie oben definiert ist.

3. C^{α}-Amid-Peptid gemäß Anspruch 1 oder Anspruch 2, **dadurch gekennzeichnet, dass** im Rest (I), C für einen für die Fmoc-SPPS-Strategie verwendbaren Arm steht.

4. C^{α}-Amid-Peptid gemäß Anspruch 3, **dadurch gekennzeichnet, dass** C für einen säurelabilen Arm steht, ausgewählt aus der Gruppe umfassend einen Rink-Arm (4-[(2,4-Dimethoxyphenyl)methyl]phenoxyacetyl), einen Arm des Wang-Typs (4-Alkoxybenzyl), einen Sieber-Arm (Xanthen-3-yloxyalkyl), einen PAL-Arm (4-2,5-Dimethoxy-alkoxybenzyl), einem 2-Chlortrityl-Arm, einen PAM-Arm (Phenylacetamidomethyl), einen SASRIN-Arm (2-Methoxy-4-Alkoxybenzyl) oder einen MBHA-Arm (4-Methyl)benzhydryl.

5. C^{α}-Amid-Peptid gemäß einem der Ansprüche 1 bis 4, **dadurch gekennzeichnet, dass** im Rest (I), D für einen für die Fmoc-SPPS-Strategie geeigneten Träger steht und vorzugsweise aus der Gruppe ausgewählt ist, umfassend ein Harz, das unter dem Namen PEGA® vermarktet wird, ein Harz, das unter dem Namen CHEMMATRIX^{Tm} vermarket wird, ein Harz des Polyacrylamid-Typs, ein Harz des Polystyroltyps oder ein gemischtes Polystyrol/Polyethylenglykol-Harz (PEG).

6. C^{α}-Amid-Peptid gemäß einem der Ansprüche 1 bis 5, **dadurch gekennzeichnet, dass** im Rest (I), n eine ganze Zahl gleich 0 ist und der Rest A die folgende Formel aufweist: 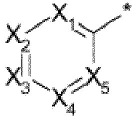 worin X₁ für C-OH steht und jedes von X₂, X₃, X₄ und X₅ wie in Anspruch 1 definiert ist.

7. C^{α}-Amid-Peptid gemäß Anspruch 6, **dadurch gekennzeichnet, dass** im Rest A, jedes von X₂, X₃ oder X₅ für CH steht und X₄ für C-R₁₁ steht, wobei R₁₁ wie in Anspruch 1 definiert ist, und vorzugsweise X₄ für CH, C-OMe oder C-NO₂ steht.

8. C^{α}-Amid-Peptid gemäß einem der Ansprüche 1 bis 5, **dadurch gekennzeichnet, dass** im Rest (I), n eine ganze Zahl gleich 0 ist und der Rest A die folgende Formel aufweist: 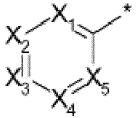 worin X₁ für ein Stickstoffatom (N) steht und jedes von X₂, X₃, X₄ und X₅ wie in Anspruch 1 definiert ist.

9. C^{α}-Amid-Peptid gemäß Anspruch 8, **dadurch gekennzeichnet, dass** im Rest A jedes von X₂, X₃ oder X₅ für CH steht und X₄ für CH oder C-R₁₁ steht, wobei R₁₁ für OCH₃ oder N(CH₃)₂ steht.

10. C^{α}-Amid-Peptid gemäß einem der Ansprüche 1 bis 5, **dadurch gekennzeichnet, dass** im Rest (I), n eine ganze Zahl gleich 0 ist und der Rest A die folgende Formel aufweist: 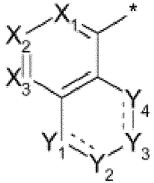 worin X₁ oder Y₄ für C-OH oder N steht, wobei das andere von X₁ oder Y₄, das nicht für C-OH oder N steht, ebenso wie jedes von X₂, X₃, Y₁, Y₂ und Y₃ wie in Anspruch 1 definiert ist.

11. C^{α}-Amid-Peptid gemäß einem der Ansprüche 1 bis 5, **dadurch gekennzeichnet, dass** im Rest (I), n eine ganze Zahl gleich 0 ist und der Rest A die folgende Formel aufweist: 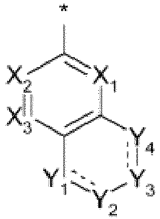 worin X₁ oder X₂ für C-OH oder N steht, wobei das andere von X₁ oder X₂, das nicht für C-OH oder N steht, ebenso wie jedes von X₃, Y₁, Y₂, Y₃ und Y₄, wie in Anspruch 1 definiert ist.

12. C^{α}-Amid-Peptid gemäß einem der Ansprüche 1 bis 11, **dadurch gekennzeichnet, dass** im Rest (I),
m für 0 oder 1 steht,
jedes von R₃, R₄, R₅, R₆ oder R₇ für H steht,
R₂ für -B-C-D steht, worin B für folgendes steht: 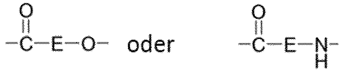 und C, D und E wie in einem der Ansprüche 1 bis 4 definiert sind.

13. C^{α}-Amid-Peptid gemäß einem der Ansprüche 1 bis 12, **dadurch gekennzeichnet, dass** im Rest (I), m gleich 0 ist und X für ein Schwefelatom (S) steht.

14. C^{α}-Amid-Peptid gemäß einem der Ansprüche 1 bis 13, **dadurch gekennzeichnet, dass** im Rest (I), R₁ eine Schutzgruppe des Schwefels ist, ausgewählt aus der Gruppe umfassend Trityl (Trt), Methyltrityl (Mtt), Methoxytrityl (MmT), Xanthenyl (Xan), Trimethoxybenzyl (Tmob), Acetamidomethyl (Acm), Trimethylacetamidomethyl (Tacm), Benzamidomethyl (Bam), Allyloxycarbonylaminomethyl (Allocam), Phthalimidomethyl (Pim), 2-(Trimethylsilyl)ethyl, t-Butyl (tBu), 2,4,6-Trimethylbenzyl, Chinolinylmethyl (Qm), Diphenyl-4-pyridylmethyl, 1-Adamantyl, Benzyloxymethyl (BOM), 2-Tetrahydropyranyl (Thp), Benzylthiomethyl, Ethylsulfenyl (SEt), t-Butylsulfenyl (StBu) Phenylsulfenyl (SPH) und 2,4-Dinitrophenyl.

15. C^{α}-Amid-Peptid gemäß einem der Ansprüche 1 bis 14, **dadurch gekennzeichnet, dass** das Peptid die allgemeine Formel (II) aufweist: 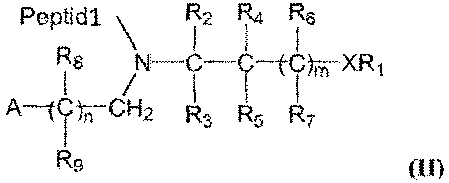 worin:
Peptid1 für eine R₁₈-(Xaa)ₖ- -Gruppe steht, worin:
k für eine ganze Zahl von 1 bis 100 steht, vorzugsweise von 4 bis 60, und noch stärker bevorzugt von 8 bis 40,
Xaa unabhängig voneinander für einen Aminosäurerest steht, der sich von einer Aminosäure der Formel H-Xaa-OH ableitet, und wenn k größer als oder gleich 2 ist, jedes Xaa mit dem benachbarten Xaa durch eine Peptidbindung verbunden ist,
R₁₈ für ein Wasserstoffatom oder einen Substituenten, des im Xaa-Rest enthaltenen N-Terminus steht,
X, m, n, A und R¹ bis R⁹ wie in Anspruch 1 definiert sind.

16. Verbindung, **dadurch gekennzeichnet, dass** sie die allgemeine Formel (I bis) aufweist: 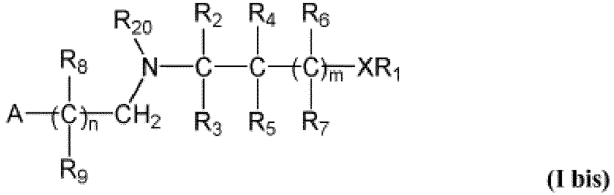 worin:
X₁, R₁, m, R₂, R₃, R₄, R₅, R₆, R₇, n, R₈, R₉ und A wie in Anspruch 1 definiert sind und R₂₀ für R₁₄ steht,
wobei R₁₄ = R₁₃ und R₁₃ für eine Schutzgruppe einer Aminogruppe steht, und vorzugsweise für eine Fmoc-Gruppe,
oder R₁₄ für einen geschützten Amino-Acyl-Rest der Formel R₁₃-Xaa- steht, wobei Xaa für einen Aminosäurerest steht, der sich von einer Aminosäure der Formel H-Xaa-OH ableitet.

17. Verbindung, **dadurch gekennzeichnet, dass** sie die allgemeine Formel (I bis) aufweist: 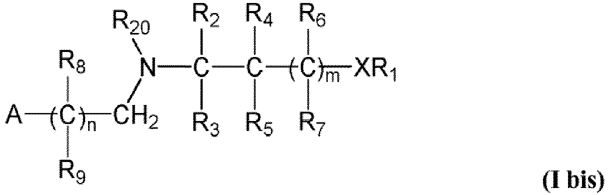 worin:
X, R₁, m, n, R₈, R₉ und A wie in Anspruch 1 definiert sind, eines von R₂, R₃, R₄, R₅, R₆ oder R₇ für den Rest -B-C-D steht, worin:
D für ein Wasserstoffatom oder einen festen Träger steht, der für die Festphasenpeptidsynthese (SPPS) geeignet ist,
C fehlt oder für einen für die SPPS verwendbaren Arm steht,
B dargestellt ist durch: 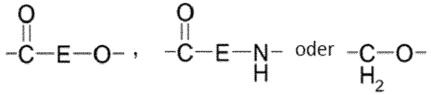 worin
E fehlt oder für eine -(Xaa)ᵢ- -Gruppe steht, worin:
i für eine ganze Zahl von 1 bis 20 steht, vorzugsweise von 1 bis 5,
jedes Xaa unabhängig voneinander für einen Aminosäurerest steht, und wenn i größer als oder gleich 2 ist, dann ist jedes Xaa mit seinem benachbarten Xaa durch eine Peptidbindung verbunden,
oder, für den Fall, dass m = 1, und R₄ oder R₅ = -B-C-D, der zweiwertige Rest B dann vorzugsweise für folgendes steht: 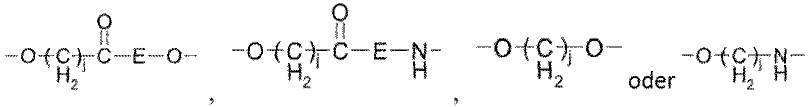 worin:
j für eine ganze Zahl von 0 bis 10 steht, vorzugsweise von 0 bis 3, und E wie oben definiert ist,
die restlichen R₂, R₃, R₄, R₅, R₆ oder R₇, die nicht für -B-C-D stehen, dann unabhängig voneinander für ein Wasserstoffatom, einen Alkylrest mit 1 bis 5 Kohlenstoffatomen, vorzugsweise einen Methylrest (-CH₃) oder einen Phenylrest (-C₆H₅) stehen, unter der Voraussetzung, dass höchstens zwei der Reste von R₂, R₃, R₄, R₅, R₆ oder R₇ gleichzeitig für Phenyl stehen,
R₂₀ für Wasserstoff oder R₁₄ steht,
wobei R₁₄ = R₁₃ und R₁₃ für eine Schutzgruppe einer Aminogruppe steht, und vorzugsweise für eine Fmoc-Gruppe,
oder R₁₄ für einen geschützten Amino-Acyl-Rest der Formel R₁₃-Xaa- steht, wobei Xaa für einen Aminosäurerest steht, der sich von einer Aminosäure der Formel H-Xaa-OH ableitet.

18. Verfahren zur Herstellung eines wie in Anspruch 15 definierten C^{α}-Amid-Peptids der Formel (II) mittels Fmoc-SPPS, **dadurch gekennzeichnet, dass** es einen Schritt umfasst, bei dem die wie in Anspruch 16 oder 17 definierte Verbindung (I bis) verlängert wird, wobei der Verlängerungsschritt das Hinzufügen von wie in Anspruch 15 definiertem Peptid1 ermöglicht.

19. Verfahren zur Herstellung eines C^{α}-Thioester-Peptids der allgemeinen Formel (IX):
Peptide1 - S - R' **(IX)**
worin:
R' für einen Rest steht, der sich von einem Thiol der Formel R'-SH ableitet, und eine Alkyl-, Aryl-, Arylalkyl-, Cycloalkyl-, Heterocycloalkyl-, Heteroarylgruppe ist, die einen oder mehrere Substituenten umfassen kann, ausgewählt aus Halogen, Carboxyl-, Sulfonat-, Ammonium-, Alkohol-, Ether-, Alkyl-, Cycloalkyl-, Heterocycloalkyl-, Aryl-, Heteroaryl-, Alkoxy-, Halogenalkyl- , Arylalkyl, Heteroarylalkyl, Arylheterocycloalkyl,
Peptid1, wie in Anspruch 15 definiert ist,
**dadurch gekennzeichnet, dass** es eine Thio-Veresterungsreaktion zwischen dem wie im Anspruch 15 definierten C^{α}-Amid-Peptid der Formel (II), in dem R₁ für H steht, und einem Thiol der Formel R'-SH umfasst,
um ein wie oben definiertes C^{α}-Thioester-Peptid der Formel (IX) und die Verbindung der Formel (I ter) zu erhalten: 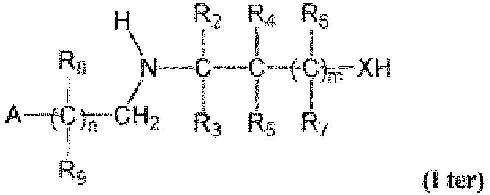
worin X, m, R₂, R₃, R₄, R₅, R₆, R₇, n, R₈, R₉ und A wie in Anspruch 1 definiert sind.

20. Verfahren zur Herstellung eines Peptids der allgemeinen Formel (X):
**Peptid1 -Yaa-Peptid2 -R₁₅** **(X)**
worin:
Peptid1 wie in Anspruch 15 definiert ist,
Yaa ein Aminosäurerest ist, der sich von einer Aminosäure der Formel H-Yaa-OH ableitet, ausgewählt aus der Gruppe umfassend Cystein, Homocystein, β-Mercapto-Valin, β-Mercapto-Leucin, β-Mercapto-Isoleucin, β-Mercapto-Phenylalanin, β-Mercapto-Lysin, β-Mercapto-Prolin, *γ*-Mercapto-Valin, *γ*-Mercapto-Isoleucin, *γ-*Mercapto-Leucin, *γ*-Mercapto-Lysin, *γ*-Mercapto-Prolin oder einer Aminosäure deren N^{α}-Stickstoffatom durch eine Gruppe substituiert ist, die eine β- oder eine *γ-*Aminothiolgruppe aufweist, wobei die Gruppe ausgewählt ist aus der Gruppe umfassend: 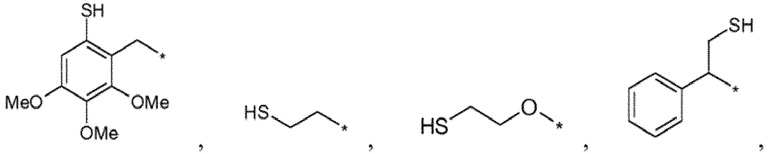 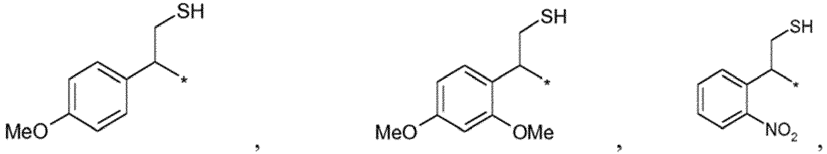 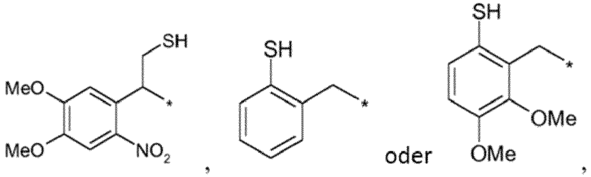
Peptid2 = (Xaa)ₗ, mit l = eine ganze Zahl von 1 bis 60, vorzugsweise von 4 bis 40,
Xaa unabhängig voneinander für einen Aminosäurerest steht, der sich von einer Aminosäure der Formel H-Xaa-OH ableitet, und wenn 1 größer als oder gleich 2 ist, dann ist jedes Xaa mit dem benachbarten Xaa durch eine Peptidbindung verbunden,
R₁₅ für -OH, NH₂ oder einen Rest der allgemeinen Formel (I) steht, worin X für ein Schwefelatom und R₁ für eine Schwefelschutzgruppe steht, die gegenüber einer Behandlung mit TFA stabil ist und unter den NCL-Bedingungen stabil ist,
**dadurch gekennzeichnet, dass** durch eine NCL-Reaktion umgesetzt wird:
ein wie in Anspruch 15 definiertes C^{α}-Amid-Peptid der allgemeinen Formel (II), in dem R₁ = H oder eine Gruppe, die unter den Bedingungen der nativen chemischen Ligation NCL labil ist,
mit einem Peptid, das einen N-terminalen β- oder *γ-*Aminothiol-Rest der allgemeinen Formel (XI) aufweist:
**H-Yaa-Peptid2 -R₁₅** **(XI)**
worin Yaa, R₁₅ und Peptid2 wie oben definiert sind, um
- das oben definierte Peptid der allgemeinen Formel (X), und
- die in Anspruch 16 oder Anspruch 17 definierte Verbindung der Formel (I bis) zu erhalten.

21. Verwendung eines wie in einem der Ansprüche 1 bis 15 definierten C^{α}-Amid-Peptids zur Herstellung eines C^{α}-Thioester-Peptids.

22. Verwendung eines wie in einem der Ansprüche 1 bis 15 definierten C^{α}-Amid-Peptids in einer nativen chemischen Ligationsreaktion NCL ("Native chemical ligations"), um ein Peptid oder ein Protein herzustellen, insbesondere eines von therapeutischem Interesse.

## Claims

1. A peptide C^{α}-amide precursor of a peptide C^{α}-thioester, **characterized in that** it comprises the radical of general formula (I): 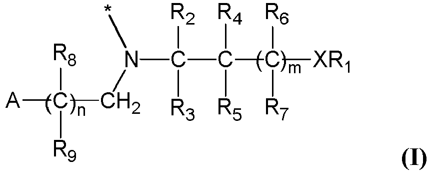 in which
X represents a sulfur or selenium atom,
R₁ represents a hydrogen atom or a protective group for the sulfur or for the selenium which is compatible with conditions of elongation by Fmoc SPPS,
m represents an integer equal to 0 or 1,
one of R₂, R₃, R₄, R₅, R₆ or R₇ represents the radical -B-C-D in which:
D represents a hydrogen atom or a solid support suitable for solid phase peptide synthesis (SPPS),
C is absent or represents an arm that can be used for SPPS,
B represents a divalent radical comprising a heteroatom,
the others of said R₂, R₃, R₄, R₅, R₆ or R₇ which do not represent -B-C-D, then represent, independently of one another, a hydrogen atom, or an alkyl radical having from 1 to 5 carbon atoms, and preferably a methyl radical (-CH₃) or a phenyl radical (-C₆H₅), on the condition that at most two of said radicals R₂, R₃, R₄, R₅, R₆ or R₇ represent at the same time a phenyl,
R₈ and R₉ represent, independently of one another, an alkyl radical having from 1 to 10 carbon atoms, and preferably a methyl radical, or a hydrogen atom,
n represents an integer ranging from 0 to 4, preferably from 0 to 1,
A representing an aryl or heteroaryl radical chosen from the group comprising
• the radical of formula: 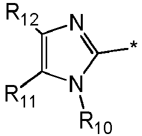 in which
R₁₀ represents an alkyl radical having from 1 to 10 carbon atoms, and preferably a methyl,
R₁₁ and R₁₂ represent, independently of one another, a hydrogen atom, a halogen atom chosen from the group comprising Cl, Br, I and F, a -CN radical, an -NO₂ radical, a -CF₃ radical, a phenyl radical (-C₆H₅), a -CONH₂ radical, an R₁₀ radical, an -OR₁₀ radical, an -SR₁₀ radical, an -N(R₁₀)₂ radical, a -COOR₁₀ radical, a -CONHR₁₀ radical or a -CON(R₁₀)₂ radical, R₁₀ being as previously defined,
• the radical of formula: 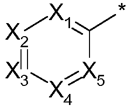 in which
at least one of X₁, X₂, X₃, X₄ and X₅ represents a nitrogen (N) atom, a C-OH radical or a C-SH radical, the others of said X₁, X₂, X₃, X₄ or X₅ which do not represent N, C-OH or C-SH, then representing, independently of one another, a C-R₁₁ radical with R₁₁ as previously defined,
• the radical of formula: 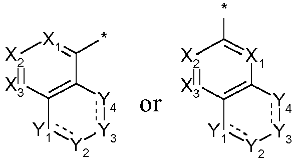 in which
at least one of X₁, X₂, X₃ and Y₄ represents a nitrogen (N) atom, a C-OH radical or a C-SH radical, the others of said X₁, X₂ or X₃ which do not represent N, C-OH or C-SH, then representing, independently of one another, a C-R₁₁ radical with R₁₁ as previously defined,
Y₁, Y₂, Y₃ and Y₄ (on condition that Y₄ does not represent C-OH or C-SH) represent, independently of one another, depending on whether they are linked via a single or double bond, a nitrogen (N) atom or an NR₁₀ group with R₁₀ as previously defined, a CH or CH₂ group, a C-R₁₁ or CHR₁₁ or CR₁₁R₁₂ group with R₁₁ and R₁₂ as previously defined, a carbonyl (C=O), an oxygen (O) atom or a sulfur (S) atom, with the condition that at most two of said Y₁, Y₂, Y₃ and Y₄ represent at the same time an oxygen atom or a sulfur atom,
one of said Y₁, Y₂, Y₃ or Y₄ possibly being absent, so as to form a 5-membered ring.

2. The peptide C^{α}-amide as claimed in claim 1, **characterized in that**, in the radical (I), the divalent radical B represents: 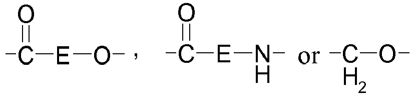 in which
E is absent or represents the -(Xaa)ᵢ- group in which:
i represents an integer ranging from 1 to 20, and preferably from 1 to 5,
each Xaa represents, independently of one another, an amino acid residue, and when i is greater than or equal to 2, each of said Xaa is connected to its neighboring Xaa via a peptide bond,
or, in the case where m = 1, and R₄ or R₅ = -B-C-D, then the divalent radical B preferably represents: 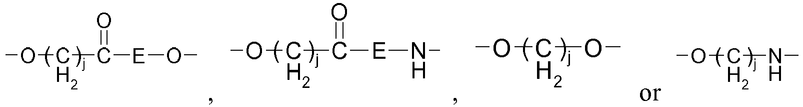 in which:
j represents an integer ranging from 0 to 10, preferably from 0 to 3, and E is as previously defined.

3. The peptide C^{α}-amide as claimed in claim 1 or claim 2, **characterized in that**, in the radical (I), C represents an arm that can be used for Fmoc SPPS.

4. The peptide C^{α}-amide as claimed in claim 3, **characterized in that** C represents an acid-labile arm chosen from the group comprising a Rink (4-[(2,4-dimethoxyphenyl)methyl]phenoxyacetyl) arm, a Wang (4-alkoxybenzyl) arm, a Sieber (xanthen-3-yloxyalkyl) arm, a PAL (4-2,5-dimethoxyalkoxybenzyl) arm, a 2-chlorotrityl arm, a PAM (phenylacetamidomethyl) arm, a SASRIN (2-methoxy-4-alkoxybenzyl) arm or an MBHA (4-methyl)benzhydryl arm.

5. The peptide C^{α}-amide as claimed in any one of claims 1 to 4, **characterized in that**, in the radical (I), D represents a solid support suitable for Fmoc SPPS and is preferably chosen from the group comprising a resin sold under the name Pega®, a resin sold under the name Chemmatrix™, a polyacrylamide resin, a polystyrene resin or a polystyrene/polyethylene glycol (PEG) mixed resin.

6. The peptide C^{α}-amide as claimed in any one of claims 1 to 5, **characterized in that**, in the radical (I), n is an integer equal to 0, and the radical A has the formula: 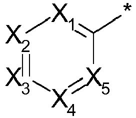 in which X₁ represents C-OH, and each of X₂, X₃, X₄ and X₅ is as defined in claim 1.

7. The peptide C^{α}-amide as claimed in claim 6, **characterized in that**, in the radical A, each of X₂, X₃ or X₅ represents CH and X₄ represents C-R₁₁ with R₁₁ as defined in claim 1, and preferably X₄ represents CH, C-OMe or C-NO₂.

8. The peptide C^{α}-amide as claimed in any one of claims 1 to 5, **characterized in that**, in the radical (I), n is an integer equal to 0, and the radical A has the formula: 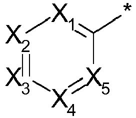 in which X₁ represents a nitrogen (N) atom and each of said X₂, X₃, X₄ and X₅ is as defined in claim 1.

9. The peptide C^{α}-amide as claimed in claim 8, **characterized in that**, in the radical A, each of X₂, X₃ or X₅ represents CH and X₄ represents CH or C-R₁₁ with R₁₁ representing OCH₃ or N(CH₃)₂.

10. The peptide C^{α}-amide as claimed in any one of claims 1 to 5, **characterized in that**, in the radical (I), n is an integer equal to 0, and the radical A has the formula: 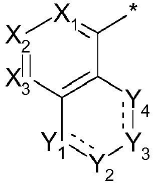 in which X₁ or Y₄ represents C-OH or N, the other said X₁ or Y₄ which does not represent C-OH or N, and also each of said X₂, X₃, Y₁, Y₂ and Y₃ are as defined in claim 1.

11. The peptide C^{α}-amide as claimed in any one of claims 1 to 5, **characterized in that**, in the radical (I), n is an integer equal to 0, and the radical A has the formula: 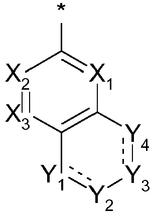 in which X₁ or X₂ represents C-OH or N, the other of said X₁ or X₂ which does not represent C-OH or N and also each of said X₃, Y₁, Y₂, Y₃ and Y₄ are as defined in claim 1.

12. The peptide C^{α}-amide as claimed in any one of claims 1 to 11, **characterized in that**, in the radical (I):
m represents 0 or 1,
each of said R₃, R₄, R₅, R₆ or R₇ represents H,
R₂ represents -B-C-D in which B represents: 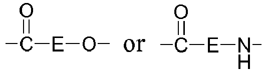
and C, D and E are as defined in any one of claims 1 to 4.

13. The peptide C^{α}-amide as claimed in any one of claims 1 to 12, **characterized in that**, in the radical (I), m is equal to 0 and X represents a sulfur (S) atom.

14. The peptide C^{α}-amide as claimed in any one of claims 1 to 13, **characterized in that**, in the radical (I), R₁ is a protective group for sulfur chosen from the group comprising trityl (Trt), methyltrityl (Mtt), methoxytrityl (Mmt), xanthenyl (Xan), tri-methoxybenzyl (Tmob), acetamidomethyl (Acm), trimethylacetamidomethyl (Tacm), benzamidomethyl (Bam), allyloxycarbonylaminomethyl (Allocam), phthalimidomethyl (Pim), 2-(trimethylsilyl)ethyl, t-butyl (tBu), 2,4,6-trimethylbenzyl, quinolinylmethyl (Qm), diphenyl-4-pyridylmethyl, 1-adamantyl, benzyloxymethyl (BOM), 2-tetrahydropyranyl (Thp), benzylthiomethyl, ethylsulfenyl (SEt), t-butylsulfenyl (StBu), phenylsulfenyl (SPh) and 2,4-dinitrophenyl.

15. The peptide C^{α}-amide as claimed in any one of claims 1 to 14, **characterized in that** said peptide has general formula (II): 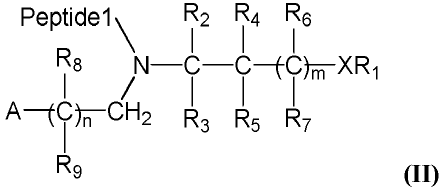 in which:
Peptide1 represents the R₁₈-(Xaa)ₖ- group in which:
k is an integer ranging from 1 to 100, preferably from 4 to 60, and even more preferentially from 8 to 40,
Xaa represents, independently of one another, an amino acid residue originating from an amino acid of formula H-Xaa-OH, and when k is greater than or equal to 2, each of said Xaa is connected to its neighboring Xaa via a peptide bond,
R₁₈ is a hydrogen atom or a substituent of the N-terminal end included in the Xaa residue,
X, m, n, A and R₁ to R₉ being as defined in claim 1.

16. A compound **characterized in that** it has general formula (Ia): 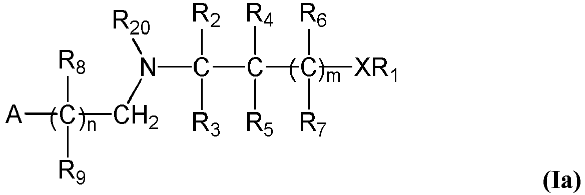 in which:
X, R₁, m, R₂, R₃, R₄, R₅, R₆, R₇, n, R₈, R₉ and A are as defined in claim 1 and R₂₀ represents R₁₄,
with R₁₄= R₁₃ and R₁₃ represents a protective group for the amine function, and preferably an Fmoc group,
or R₁₄ represents a protected aminoacyl residue of formula R₁₃-Xaa- with Xaa representing an amino acid residue originating from an amino acid of formula H-Xaa-OH.

17. A compound **characterized in that** it has general formula (Ia): 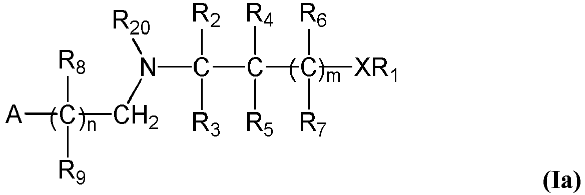 in which:
X, R₁, m, n, R₈, R₉ and A are as defined in claim 1,
one of R₂, R₃, R₄, R₅, R₆ or R₇ represents the radical -B-C-D in which:
D represents a hydrogen atom or a solid support suitable for solid phase peptide synthesis (SPPS),
C is absent or represents an arm that can be used for SPPS,
B represents: 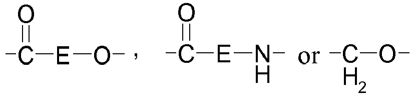 in which
E is absent or represents the -(Xaa)ᵢ- group in which:
i represents an integer ranging from 1 to 20, and preferably from 1 to 5,
each Xaa represents, independently of one another, an amino acid residue, and when i is greater than or equal to 2, each of said Xaa is connected to its neighboring Xaa via a peptide bond,
or, in the case where m = 1, and R₄ or R₅ = -B-C-D, then the divalent radical B preferably represents: 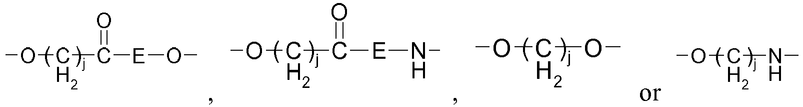
in which:
j represents an integer ranging from 0 to 10, preferably from 0 to 3, and E is as previously defined,
the others of said R₂, R₃, R₄, R₅, R₆ or R₇ which do not represent -B-C-D, then represent, independently of one another, a hydrogen atom, or an alkyl radical having from 1 to 5 carbon atoms, and preferably a methyl radical (-CH₃) or a phenyl radical (-C₆H₅), on the condition that at most two of said radicals R₂, R₃, R₄, R₅, R₆ or R₇ represent at the same time a phenyl,
R₂₀ represents a hydrogen or R₁₄,
with R₁₄= R₁₃ and R₁₃ represents a protective group for the amine function, and preferably an Fmoc group,
or R₁₄ represents a protected aminoacyl residue of formula R₁₃-Xaa- with Xaa representing an amino acid residue originating from an amino acid of formula H-Xaa-OH.

18. A process for preparing a peptide C^{α}-amide of formula (II) as defined in claim 15 by Fmoc SPPS, **characterized in that** it comprises a step of elongation of the compound (Ia) as defined in claim 16 or claim 17, said elongation step making it possible to add Peptide1 as defined in claim 15.

19. A process for preparing a peptide C^{α}-thioester of general formula (IX):
Peptide1-S-R' **(IX)**
in which:
R'- represents a radical originating from a thiol of formula R'-SH, and is an alkyl, aryl, arylalkyl, cycloalkyl, heterocycloalkyl or heteroaryl group as defined above, it being possible for each of said groups to also comprise one or more conventional substituents chosen from: halogen, carboxyl, sulfonate, ammonium, alcohol, ether, alkyl, cycloalkyl, heterocycloalkyl, aryl, heteroaryl, alkoxy, haloalkyl, arylalkyl, heteroarylalkyl and arylheterocycloalkyl,
Peptide1 is as defined in claim 15,
**characterized in that** it comprises a reaction for thioesterification between the peptide C^{α}-amide of formula (II) as defined in claim 15, in which R₁ is equal to H, and a thiol of formula R'-SH,
in order to obtain the peptide C^{α}-thioester of formula (IX) as defined above, and the compound of formula (Ib): 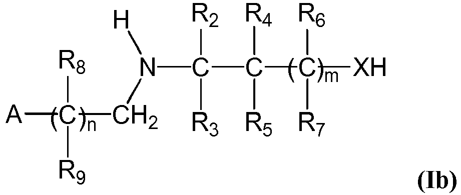
in which X, m, R₂, R₃, R₄, R₅, R₆, R₇, n, R₈, R₉ and A are as defined in claim 1.

20. A process for preparing a peptide of general formula (X):
Peptide1-Yaa-Peptide2-R₁₅ **(X)**
in which:
Peptide1 is as defined in claim 15,
Yaa is an amino acid residue originating from an amino acid of formula H-Yaa-OH chosen from the group comprising a cysteine, a homocysteine, a (β-mercaptovaline, a β-mercaptoleucine, a (β-mercaptoisoleucine, a (β-mercaptophenylalanine, a β mercaptolysine, a (β-mercaptoproline, a γ-mercaptovaline, a γ-mercaptoisoleucine, a γ-mercaptoleucine, a γ-mercaptolysine, a γ-mercaptoproline, or an amino acid substituted on its nitrogen atom N^{α} with a group containing a β- or γ-aminothiol function, said group being chosen from the group comprising: 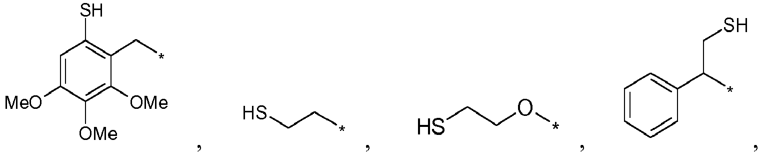 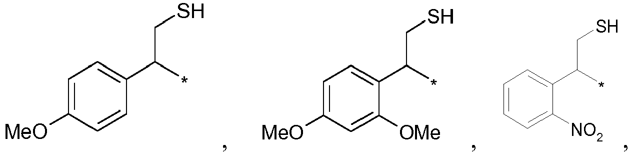 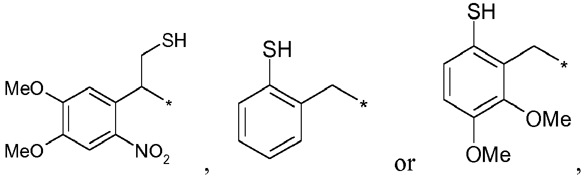
Peptide2 = (Xaa)₁, with 1 = an integer ranging from 1 to 60, preferably from 4 to 40, Xaa represents, independently of one another, an amino acid residue originating from an amino acid of formula H-Xaa-OH, and when 1 is greater than or equal to 2, each of said Xaa is connected to its neighbor Xaa via a peptide bond,
R₁₅ represents -OH, -NH₂ or a radical of general formula (I) in which X represents a sulfur atom and R₁ is a protective group for sulfur which is stable with respect to a treatment with TFA and stable under NCL conditions,
**characterized in that**:
a peptide C^{α}-amide of general formula (II) as defined in claim 15, in which R₁ = H or a group which is labile under NCL native chemical ligation conditions, is reacted, by means of an NCL reaction,
with a peptide possessing an N-terminal β- or γ-aminothiol residue of general formula (XI):
H-Yaa-Peptide2-R₁₅ **(XI)**
in which Yaa, R₁₅ and Peptide2 are as defined above,
in order to obtain
- the peptide of general formula (X) as defined above and
- the compound of formula (Ia) as defined in claim 16 or claim 17.

21. The use of a peptide C^{α}-amide as defined in any one of claims 1 to 15, for preparing a peptide C^{α}-thioester.

22. The use of a peptide C^{α}-amide as defined in any one of claims 1 to 15, in an NCL native chemical ligation reaction for preparing a peptide or a protein, in particular of therapeutic interest.


### SEQUENCE LISTING

<110> CNRS
<120> Peptides C -amides, leurs procédés de préparation et leurs utilisations comme précurseurs de peptides C -thioesters pour la synthèse de protéines
<130> BCT120455IMA
<140> PCT/FR2012/053074
   <141> 2012-12-21
<150> FR12 50060
   <151> 2012-01-03
<160> 6
<170> PatentIn version 3.5
<210> 1
   <211> 5
   <212> PRT
   <213> Artificial Sequence
<220>
   <223> groupe B
<400> 1
   Leu Tyr Arg Ala Gly
   1               5
<210> 2
   <211> 6
   <212> PRT
   <213> Artificial Sequence
<220>
   <223> peptidyle résine 2
<400> 2
   Cys Leu Tyr Arg Ala Gly
   1               5
<210> 3
   <211> 4
   <212> PRT
   <213> Artificial Sequence
<220>
   <223> peptide 1
<400> 3
   Tyr Arg Phe Gly
   1
<210> 4
   <211> 10
   <212> PRT
   <213> Artificial Sequence
<220>
   <223> peptide 9
<400> 4
   Tyr Arg Phe Gly Cys Leu Tyr Arg Ala Gly
   1 5 10
<210> 5
   <211> 18
   <212> PRT
   <213> Artificial Sequence
<220>
   <223> peptide 1
<400> 5
   Ala Ser Cys Asn Gly Val Cys Pro Phe Glu Met Pro Pro Cys Gly Thr
   1 5 10 15
   Ser Ala
<210> 6
   <211> 17
   <212> PRT
   <213> Artificial Sequence
<220>
   <223> peptide 16
<400> 6
   Cys Arg Cys Ile Pro Val Gly Leu Ile Gly Tyr Cys Arg Asn Pro Ser
   1 5 10 15
   Gly